# EUROPEAN PATENT APPLICATION

(11) **EP 2 338 877 A1**
(43) Date of publication of application: **29.06.2011**
(21) Application number: 09179351.3
(22) Date of filing: 15.12.2009
(51) Int. Cl.: C07D 205/08, C07D 409/04, A61K 31/397, A61K 31/381, A61P 35/00

(54) **Combretastatin Derivatives and Uses Therefor**

(71) Applicant: The Provost, Fellows and Scholars of the College of the Holy and Undivided Trinity of Queen Elizabeth near Dublin, Dublin 2 (IE)
(72) Inventor: Meegan, Mary J., 16, Dublin (IE); Zisterer, Daniela, Bray, Co. Wicklow (IE); Carr, Miriam, Co. Limerick (IE); Greene, Thomas, Co. Tipperary (IE); O'Boyle, Niamh, Co. Kildare (IE); Greene, Lisa, 15, Dublin (IE)
(74) Representative: Lane, Cathal Michael

(57) **Abstract**

Provided herein are synthetic derivatives of combretastatin A-4 in which the aromatic rings are locked into a non-isomerisable active conformation, thus resulting in improved, stable compounds. The novel compounds are structurally related to combretastatin A-4 (CA-4) and lock the rings into the known active conformation by means of a four membered nitrogen containing heterocyclic ring, such as a beta-lactam ring, incorporated into the standard CA-4 structure. The compounds exhibit potent anti-cancer activity.

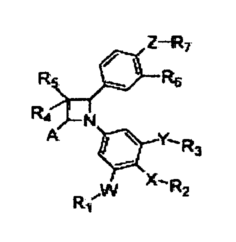

## Description

### Field of the Invention

The present invention relates to synthetic derivatives of combretastatin A-4 in particular those in which the aromatic rings are locked into a non-isomerisable active conformation, thus resulting in improved, stable compounds. Of particular interest in the present invention are compounds showing anti-cancer activity.

### Background to the Invention

Cancer is one of the major causes of death worldwide. Although many advances have been made in the treatment and management of the disease, the existence of chemotherapy-resistance means there is still a great need to develop new strategies and drugs for its treatment. The economic impact of cancer can be measured in terms of the cost of in-patient and out-patient hospital treatment, counselling for cancer-sufferers and their families and loss of earnings for patients and those who care for them at home. Therefore, the development of new treatments present a major socio-economic challenge.

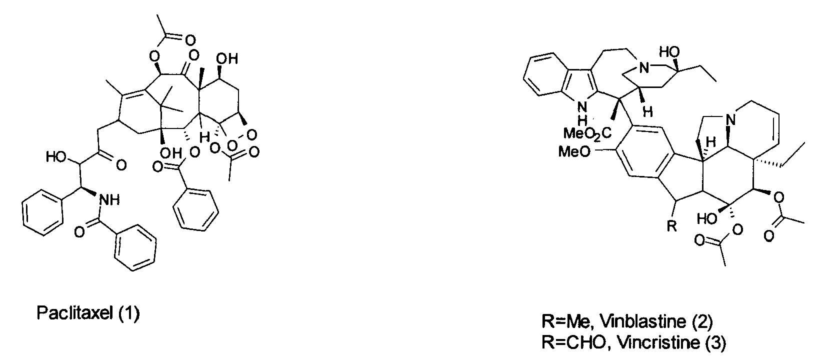

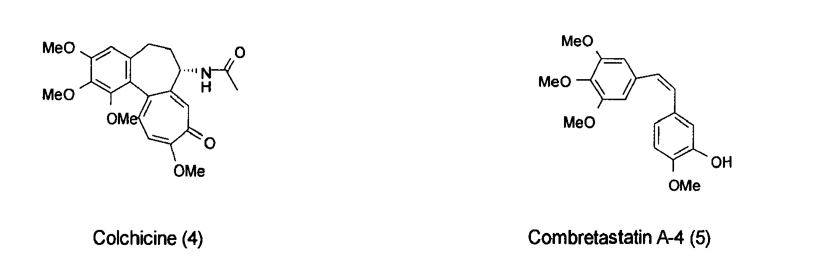

Microtubules represent one of the most effective cancer targets identified to date. Microtubules are key components of the cytoskeleton, and are composed of long filamentous tubular protein polymers, which are essential for all eukaryotic cells. In particular they are crucial in the maintenance and development of cell shape, in the transport of cellular components such as mitochondria and vesicles in cells, in cell signalling processes and in mitosis and cell division. Their importance in mitosis and cell division make microtubules effective targets for anticancer drugs.

Microtubules and their dynamic processes have become the target for a diverse range of antimitotic drugs, each group with a characteristic identified binding site. The three charcterised binding sites of tubulin are the taxane domain, the vinca domain, and the colchicine domain and many compounds interact with tubulin at these known sites.

Among the most successful of the microtubule targeting chemotherapeutic agents are:
- paclitaxel (1), which binds to tubulin at the taxane site;
- the Vinca alkaloids vinblastine (2) and vincristine (3), which bind at the vinca domain; and
- colchicine (4), which binds the colchicine domain,
all resulting in depolymerisation of microtubules and destruction of mitotic spindles at high concentrations which affect microtubule dynamics.

The Combretastatins are a family of stilbene type natural products derived from *Combretum caffrum,* a South African tree, which have been shown to be tubulin binding agents partly resembling colchicine in structure. Drugs that bind to the colchicine domain are undergoing extensive investigation as vascular targeting agents (VTAs) for cancer chemotherapy. Tubulin binding agents have both antimitotic and antivascular effects that lead to inhibition of spindle formation (mitosis arrest) and reduced tumour blood flow respectively. The small molecule VTAs known to date are generally microtubule destabilising agents. The strategy behind microtubule destabilising agents is to disrupt rapidly proliferating and immature tumour endothelium based on their reliance on a tubulin cytoskeleton to maintain their cell shape.

In contrast to colchicine the anti-vascular effects of combretastatin A4 [CA-4] (5) *in vivo* are apparent well below the maximum tolerated dose, offering a wide therapeutic window. CA-4 as well as being a potent inhibitor of colchicine binding is also shown to inhibit the growth and development of blood vessels. However, the low solubility and high lipophilicity of CA-4 make it particularly unattractive from a formulation perspective. The disodium phosphate salt of CA-4 (CA-4P) has much improved solubility and is currently in clinical trials for the treatment of thyroid cancer. It is in itself inactive but there is rapid phosphate hydrolysis *in vivo* by endogenous nonspecific phosphatases under physiological conditions to produce CA-4. The clinical use of CA-4P may be hindered by instability, toxicity, drug resistance and limited bioavailability.

Only the *cis* configuration of CA-4 is biologically active, with the trans form showing little or no activity. CA-4 can readily isomerise to an inactive *trans* stilbene configuration hindering its use therapeutically. In particular, there is notable predilection for photochemical isomerisation to the inactive *trans* isomer.

Accordingly, a number of synthetic CA-4 analogues have been prepared in order to mitigate the problematic isomerisation of the cis-double bond in CA-4. The synthetic analogues invariably comprises rigid ring structures locking the CA-4 rings in to the desired relative stereochemistry. A selection of the synthetic analogues shown to be capable of binding to and depolymerising tubulin are shown below:

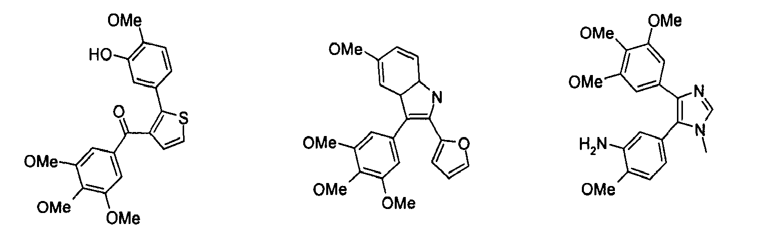

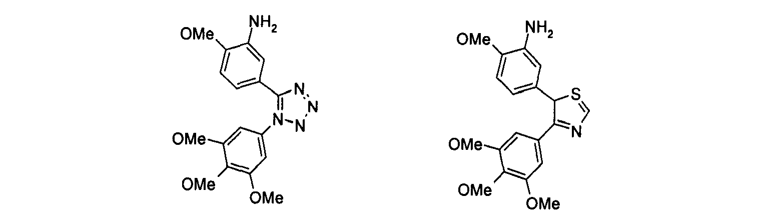

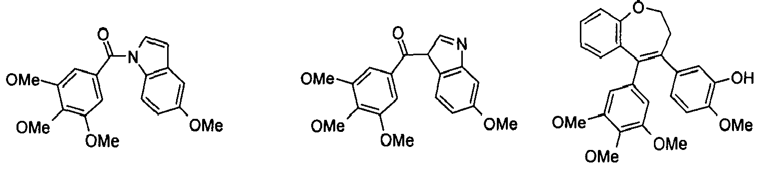

Sun et al. (Bioorg. Med. Chem. Lett. 2004, 14, 2041-2046) disclose CA-4 1,4-bisarylazetidinone analogues substituted at C3 with hydroxy, methoxy and acetoxy groups.

VTAs represent another important class of therapeutics in the treatment of cancer. For example, Avastin (bevacizumab) is an anti-vascular endothelial growth factor (VEGF) monoclonal antibody first approved for marketing in 2004. The drug is used to treat metastatic colon carcinoma and as a first-line treatment for advanced, metastatic or recurrent non-small cell lung cancer. It is currently in registration for the first-line treatment of metastatic breast cancer and metastatic renal cell carcinoma and late stage clinical trials for the treatment of ovarian cancer, gastrointestinal stromal tumors (GIST), prostate cancer, pancreatic cancer, melanoma, glioblastoma multiforme and multiple myeloma. However, a small molecule VTA would be more attractive from a purification point of view.

The cathepsin proteases are a family of protease enzymes over-expressed in tumour cells. They are translocated to cell membrane or secreted from tumour cells where they participate in the degradation of components of extracellular matrix facilitating tumour cell invasion, angiogenesis and metastasis of the cancer. Small molecules targeting cathepsins have thus been identified as promising therapeutic targets for development of new anti-tumour drugs.

A series of molecules having a dual pharmacophore targeting both tubulin polymerisation and cathepsin proteases have yet to be reported.

Accordingly, and notwithstanding the state of the art, it would be desirable to provide alternative compounds showing promising activity against tumour cell lines. Desirably, such compounds would be conformationally locked derivatives of CA-4 incapable of isomerising to the inactive *trans* configuration.

### Summary of the Invention

The present invention discloses the synthesis of a family of nitrogen containing heterocyclic compounds which function as antitumour agents over a comprehensive range of tumour cell lines at nanomolar concentrations.

The novel compounds are structurally related to combretastatin A-4 (CA-4) and lock the rings into the known active conformation by means of a four membered nitrogen containing heterocyclic ring, such as a ß-lactam ring, incorporated into the standard CA-4 structure. It is envisaged that the overall conformation of the molecule may allow it to interact with the tubulin binding site, for example it is thought that the rigid heterocyclic ring gives the molecule the correct dihedral angle to interact with the tubulin binding site. An additional advantage of these conformationally restricted compounds is that the stilbene cis/trans isomerism observed with CA-4, for example in heat, light and protic media is eliminated.

These novel analogues potently inhibit the growth of human cancer cells including breast carcinoma MCF-7 cells, human chronic myeloid leukaemia K562 cells and human promyelocytic leukaemia HL-60 cells, for example as demonstrated in an MTT cell viability assay. Compounds of the invention may exhibit IC50 values in the nanomolar or subnanomolar range.

The novel compounds may also inhibit cathepsin activity therefore these compounds may not only target the tumour directly by inhibiting tubulin but in addition may prevent angiogenesis and prevent the formation of metastases. These compounds could be useful in the treatment of many cancers including breast cancer.

Accordingly, in a first aspect the present invention provides for a compound of the general formula: 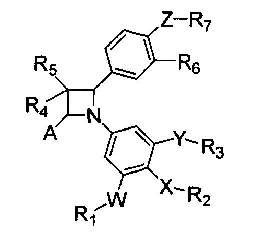 a tautomer thereof, a pharmaceutically acceptable salt thereof, or a hydrate thereof,
wherein W, X, Y and Z may be the same or different and may be selected from the group consisting of CH₂, O, S and NH;
R₁ to R₃ and R₇ may be the same or different and may be C₁-C₅ alkyl;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from H, =O, =S;
R₄ may be selected from the group consisting of hydrogen, hydroxy, amino, and halogen; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy;
such that when R₅ is hydrogen R₄ is not hydroxy.

As used herein, the term "Cₓ₋C_{y} alkyl" embraces Cₓ-C_{y} unbranched alkyl, Cₓ-C_{y} branched alkyl and combinations thereof. The term (cyclo)alkyl does not preclude the presence of one or more C-C unsaturated bonds in the carbon (ring)/chain unless otherwise indicated. The terms aryl and heteroaryl encompass both non-fused and fused aromatic and non-fused and fused heteroaromatic rings respectively. The term aliphatic embraces unbranched aliphatic, branched aliphatic, and combinations thereof. The term (cyclo)aliphatic does not preclude the presence of one or more C-C unsaturated bonds in the carbon (ring)/chain unless otherwise indicated.

Advantageously, compounds of the present invention exhibit low toxicity. For example, the compounds show low toxicity to normal mammary epithelial cells. Further advantageously, compounds of the present invention may exhibit half-lives of greater than 24 hours in human plasma.

W, X, Y and Z may be the same or different and may be selected from the group consisting of O, S and NH. W, X, Y and Z may be the same or different and may be selected from the group consisting of O, and S. W, X, Y and Z may be O. R₁ to R₃ and R₇ may be Me. W, X, Y and Z may be O and R₁ to R₃ and R₇ may be Me.

R₆ may be selected from the group consisting of hydrogen, amino, hydroxy, OC(O)R₈, SC(O)R₈, NC(O)R₈, OP(O)(OR₉)(OR₁₀) and combinations thereof. R₆ may be selected from the group consisting of amino, hydroxy, OC(O)R₈, NC(O)R₈, OP(O)(OR₉)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR₁₀).

As used herein the terms metal cations and polyatomic cations refer to pharmaceutically acceptable cations. Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

As will be appreciated by a person skilled in the art esters, thioesters, amides and phosphonates of the respective formulae OC(O)R₈, SC(O)R₈, NC(O)R₈, OP(0)(ORg)(OR₁₀) may be cleaved *in-vivo* by esterases, thioesterases, proteases and phosphatases to yield free hydroxy, thiol and amino groups.

A may be selected from =O and =S. A may be =O. R₄ may be selected from the group consisting of hydrogen, and halogen. R₄ may be hydrogen.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of ―CH=CH_{2,} phenyl, 4-hydroxyphenyl, 4-aminophenyl and thienyl.

The compound of the present invention may take the general formula: 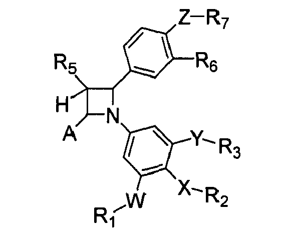 a tautomer thereof, a pharmaceutically acceptable salt thereof, or a hydrate thereof,
wherein W, X, Y and Z are the same or different and are selected from the group consisting of O, S and NH;
R₁ to R₃ and R₇ are the same or different and are C₁-C₅ alkyl;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from =O and =S; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

W, X, Y and Z may be the same or different and may be selected from the group consisting of O and S. W, X, Y and Z may be O. R₁ to R₃ and R₇ may be Me. W, X, Y and Z may be O and R₁ to R₃ and R₇ may be Me.

R₆ may be selected from the group consisting of hydrogen, amino, hydroxy, OC(O)R₈, SC(O)R₈, NC(O)R₈, OP(O)(OR₉)(OR₁₀) and combinations thereof. R₆ may be selected from the group consisting of amino, hydroxy, OC(O)R₈, NC(O)R₈, OP(O)(OR₉)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR₁₀).

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

A may be selected from =O and =S. A may be =O.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of -CH=CH₂, phenyl, 4-hydroxyphenyl, 4-aminophenyl and thienyl.

The compound of the present invention may take the general formula: 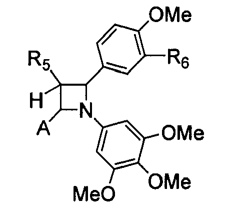 wherein A may be selected from =O and =S;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

R₆ may be selected from the group consisting of hydrogen, amino, hydroxy, OC(O)R₈, SC(O)R₈, NC(O)R₈, OP(O)(OR₉)(OR₁₀) and combinations thereof. R₆ may be selected from the group consisting of amino, hydroxy, OC(O)R₈, NC(O)R₈, OP(O)(OR₉)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR₁₀).

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of-CH=CH₂, phenyl, 4-hydroxyphenyl, 4-aminophenyl and thienyl.

The compound of the present invention may take the general formula: 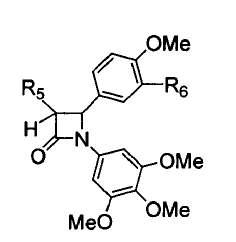 wherein R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations; and
R₅ may be selected from the group consisting of hydrogen, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

R₆ may be selected from the group consisting of hydrogen, amino, hydroxy, OC(O)R₈, SC(O)R₈, NC(O)R₈, OP(O)(OR₉)(OR₁₀) and combinations thereof. R₆ may be selected from the group consisting of amino, hydroxy, OC(O)R₈, NC(O)R₈, OP(O)(OR₉)(OR₁₀) and combinations thereof. R₆ may be selected from the group consisting of hydroxy, OC(O)R₈, OP(0)(ORg)(OR₁₀) and combinations thereof. R₆ may be selected from the group consisting of hydroxy and OP(O)(OR₉)(OR₁₀)

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of -CH=CH₂, phenyl, 4-hydroxyphenyl, 4-aminophenyl and thienyl.

The compound of the present invention may take the general formula: 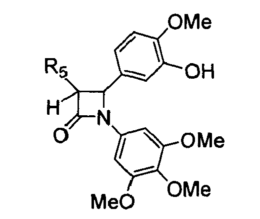 R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of -CH=CH₂, phenyl, 4-hydroxyphenyl, 4-aminophenyl and thienyl.

The compounds of the present invention may be the racemic [denoted (±)] *trans* isomer. As used here the *trans* isomer is the isomer in which the R₅ substituent at C3 on the ring and the aryl substituent at C4 of the ring are disposed in a *trans* or *anti* relationship (see below). Where R₅ is a small alkyl substituent such as Me the compound may be the racemic *cis* isomer, *i.e.* the R₅ substituent at C3 on the ring and the aryl substituent at C4 of the ring are disposed in a *cis* or *syn* relationship.

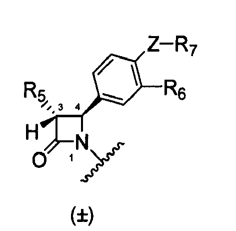

In a further aspect the present invention provides for a substantially enantiopure molecule of the of the general formula: 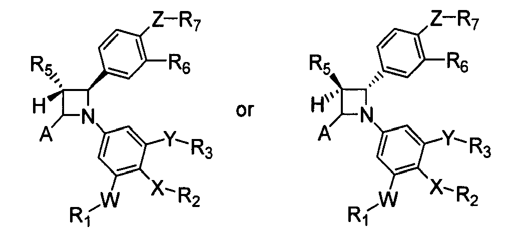 a tautomer thereof, a pharmaceutically acceptable salt thereof, or a hydrate thereof,
wherein W, X, Y and Z may be the same or different and may be selected from the group consisting of CH₂, O S and NH;
R₁ to R₃ and R₇ may be the same or different and may be C₁-C₅ alkyl;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from =0 and =S; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

W, X, Y and Z may be the same or different and may be selected from the group consisting of O S and NH. W, X, Y and Z may be the same or different and may be selected from the group consisting of O and S. W, X, Y and Z may be O. R₁ to R₃ and R₇ may be Me. W, X, Y and Z may be O and R₁ to R₃ and R₇ may be Me.

R₆ may be selected from the group consisting of hydrogen, amino, hydroxy, OC(O)R₈, SC(O)R₈, NC(O)R₈, OP(O)(OR₉)(OR₁₀) and combinations thereof. R₆ may be selected from the group consisting of amino, hydroxy, OC(O)R₈, NC(O)R₈, OP(O)(OR₉)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR₁₀).

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of -CH=CH₂, phenyl, 4-hydroxyphenyl, 4-aminophenyl and thienyl.

The substantially enantiopure molecule may be of the general formula: 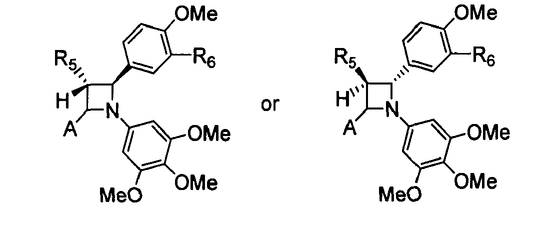 a tautomer thereof, a pharmaceutically acceptable salt thereof, or a hydrate thereof,
wherein A may be selected from =O and =S;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

R₆ may be selected from the group consisting of hydrogen, amino, hydroxy, OC(O)R₈, SC(O)R₈, NC(O)R₈, OP(O)(ORg)(OR₁₀) and combinations thereof. R₆ may be selected from the group consisting of amino, hydroxy, OC(O)R₈, NC(O)R₈, OP(O)(ORg)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR₁₀).

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of ―CH=CH₂, phenyl, 4-hydroxyphenyl, 4-aminophenyl and thienyl.

The substantially enantiopure molecule may be of the general formula: 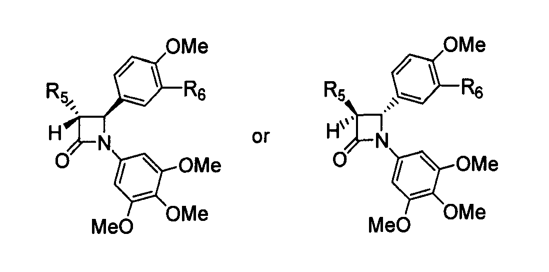 a tautomer thereof, a pharmaceutically acceptable salt thereof, or a hydrate thereof,
wherein R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

R₆ may be selected from the group consisting of hydrogen, amino, hydroxy, OC(O)R₈, SC(O)R₈, NC(O)R₈, OP(O)(ORg)(OR₁₀) and combinations thereof. R₆ may be selected from the group consisting of amino, hydroxy, OC(O)R₈, NC(O)R₈, OP(O)(ORg)(OR₁₀) and combinations thereof. R₆ may be selected from the group consisting of hydroxy, OC(O)R₈, OP(O)(OR₉)(OR₁₀) and combinations thereof. R₆ may be selected from the group consisting of hydroxy and OP(O)(OR₉)(OR)₁₀.

Suitable metal cations include calcium, magnesium, potassium, silver, sodium, zinc and combinations thereof. Suitable polyatomic cations include benzathine, cholinate, diethanolamine, diethylamine, lysine, meglumine, piperazine, tromethamine and combinations thereof.

R₅ may be selected from the group consisting of hydrogen, halogen, C₁-C₁₀ aliphatic having no C-C unsaturated bond in the chain, and C₃-C₂₀ cycloaliphatic having no C-C unsaturated bond in the ring. R₅ may be selected from the group consisting of hydrogen, chloro, Me and Et.

R₅ may be a moiety having C-C unsaturated bonds. The moiety having C-C unsaturated bonds can be aliphatic or aromatic. For example, R₅ may be selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

R₅ may be selected from the group consisting of C₂-C₁₀ aliphatic having at least one C-C unsaturated bond in the chain, C₅-C₁₀ aryl, C₃-C₁₀ heteroaryl, C₅-C₁₀ aryloxy, C₃-C₁₀ heteroaryloxy, C₂-C₅ alkoxy having C-C unsaturated bonds in the alkyl chain and combinations thereof, optionally substituted one or more times with at least one of hydroxy, amino, halogen, cyano and C₁-C₅ alkoxy.

R₅ may be selected from the group consisting of C₂-C₁₀ aliphatic having at least one C-C unsaturated bond in the chain, C₅-C₁₀ aryl, C₃-C₁₀ heteroaryl, -OCH=CH₂ and combinations thereof, optionally substituted one or more times with at least one of hydroxy, amino, halogen, and cyano.

R₅ may be selected from the group consisting of C₂-C₁₀ aliphatic having at least one C-C unsaturated bond in the chain, C₅-C₁₀ aryl, C₃-C₁₀ heteroaryl, and combinations thereof, optionally substituted one or more times with at least one of hydroxy, amino, halogen, and cyano.

R₅ may be selected from the group consisting of C₂-C₅ aliphatic having at least one C-C unsaturated bond in the chain, phenyl, thienyl and combinations thereof, optionally substituted one or more times with at least one of hydroxy, amino, halogen, cyano and C₁-C₅ alkoxy.

R₅ may be selected from the group consisting of -CH=CH₂, phenyl, 4-hydroxyphenyl, 4-aminophenyl and thienyl.

In a further aspect the present invention provides for a pharmaceutical composition comprising a compound according to the present invention, a tautomer thereof, a pharmaceutically acceptable salt thereof, or a hydrate thereof together with a pharmaceutical acceptable carrier or excipient.

In yet a further aspect the present invention provides for a compound according to the present invention, a tautomer thereof, a pharmaceutically acceptable salt thereof, or a hydrate thereof for use in the treatment of a disorder that involves vascular proliferation.

The disorder that involves vascular proliferation may be a cancer. The cancer may be a metastatic cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer. The invention further provides for a compound according to the present invention, a tautomer thereof, a pharmaceutically acceptable salt thereof, or a hydrate thereof for the inhibition of tubulin formation.

In yet a further aspect the invention provides for a compound according to the present invention, a tautomer thereof, a pharmaceutically acceptable salt thereof, or a hydrate thereof for use in the treatment of a disorder associated with cathepsin protease activity.

The cathepsin protease may be selected from the group consisting of cathepsin K and cathepsin L. The disorder associated with cathepsin protease activity may be a cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer.

The present invention also provides for a method of treating a disorder that involves vascular proliferation in a patient, comprising administering to the patient suffering therefrom a pharmaceutically effective amount of a compound according to the present invention, a tautomer thereof, a pharmaceutically acceptable salt thereof, or a hydrate thereof.

The disorder that involves vascular proliferation may be a cancer. The cancer may be a metastatic cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer.

In yet a further aspect the present invention provides for a method of treating a disorder associated with cathepsin protease activity in a patient, comprising administering to the patient a pharmaceutically effective amount of a compound according to the present invention, a tautomer thereof, a pharmaceutically acceptable salt thereof, or a hydrate thereof.

The cathepsin protease may be selected from the group consisting of cathepsin K and cathepsin L. The disorder associated with cathepsin protease activity may be a cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer.

In yet a further aspect the present invention provides for use of a compound according to the present invention, a tautomer thereof, a pharmaceutically acceptable salt thereof, or a hydrate thereof in the manufacture of a medicament for the treatment of a disorder that involves vascular proliferation.

The disorder that involves vascular proliferation may be a cancer. The cancer may be a metastatic cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer.

The invention further provides for the use of a compound according to the present invention, a tautomer thereof, a pharmaceutically acceptable salt thereof, or a hydrate thereof in the manufacture of a medicament for the inhibition of tubulin formation.

In yet a further aspect the invention provides for the use of a compound according to the present invention, a tautomer thereof, a pharmaceutically acceptable salt thereof, or a hydrate thereof in the manufacture of a medicament for the treatment of a disorder associated with cathepsin protease activity.

The cathepsin protease may be selected from the group consisting of cathepsin K and cathepsin L. The disorder associated with cathepsin protease activity may be a cancer. The cancer may be selected from the group consisting of leukaemia, lymphoma, non-small cell lung cancer, colon, central nervous system, melanoma, ovarian, renal, prostate, pancreatic, liver, bone, cervical and breast cancer. The cancer may be breast cancer.

The compounds of the present invention may be found or isolated in the form of prodrugs, tautomers, esters, salts, hydrates or solvates - all of which are embraced by the present invention.

Where suitable, it will be appreciated that all optional and/or preferred features of one embodiment of the invention may be combined with optional and/or preferred features of another/other embodiment(s) of the invention.

### Brief Description of the Drawings

Additional features and advantages of the present invention are described in, and will be apparent from, the detailed description of the invention and from the drawings in which:

**Figure 1** illustrates docking of β-lactam compound **14** in the active site of cathepsin K;

**Figure 2** illustrates that compound **14** inhibits purified human liver cathepsin-L activity; and

**Figure 3** illustrates that CA4 and Compound **4** potently induce apoptosis in *ex-vivo* chronic myeloid leukaemia patient samples.

### Detailed Description of the Invention

It should be readily apparent to one of ordinary skill in the art that the examples disclosed herein below represent generalised examples only, and that other arrangements and methods capable of reproducing the invention are possible and are embraced by the present invention.

All reagents were commercially available and were used without further purification unless otherwise indicated. IR spectra were recorded as thin films on NaCl plates or as KBr discs on a Perkin-Elmer Paragon 100 FT-IR spectrometer. ¹H and ¹³C NMR spectra were obtained on a Bruker Avance DPX 400 instrument at 20°C, 400.13MHz for ¹H spectra, 100.61 MHz for ¹³C spectra, in either CDCl₃, CD₃COCD₃ or CD₃OD (internal standard tetramethylsilane). Low resolution mass spectra were run on a Hewlett-Packard 5973 MSD GC-MS system in an electron impact mode, while high resolution accurate mass determinations for all final target compounds were obtained on a Micromass Time of Flight mass spectrometer (TOF) equipped with electrospray ionization (ES) interface operated in the positive ion mode at the High Resolution Mass Spectrometry Laboratory. Thin layer chromatography was performed using Merck Silica gel 60 TLC aluminium sheets with fluorescent indicator visualizing with UV light at 254nm. Flash chromatography was carried out using standard silica gel 60 (230-400 mesh) obtained from Merck. All products isolated were homogenous on TLC.

### General preparation of Compounds

The first step in the synthesis of the β-lactams was the formation of the imine precursors. This is achieved by reaction of the appropriately substituted benzaldehydes and anilines in a simple one-step reflux (Scheme 1). A solution of the appropriately substituted aryl aldehyde (0.1 mol) and the appropriately substituted aryl amine (0.1 mol) in ethanol (50 ml) was heated to reflux for three hours. The reaction mixture was reduced to 25 ml under vacuum, and the solution transferred to a beaker. The mixture was left to stand and the Schiff base product crystallized out of the solution. The crude product was then re-crystallized from ethanol and filtered to yield the purified product.

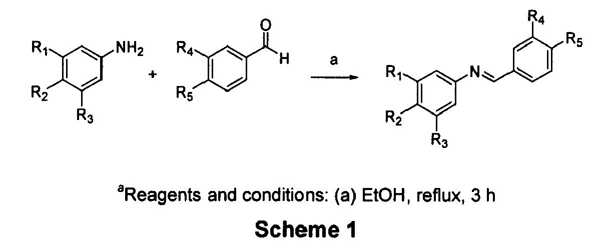

In cases where a free hydroxy group was present on the benzaldehyde, this was firstly protected using the trimethyldibutylsilyl group. The TBDMS ether is one of the most popular silyl protective groups due to its easy introduction, stability under a variety of reactions, and easy removal under conditions that do not attack other functional groups. It was more difficult to form imines from ketone precursors such as acetophenone and benzophenone. The use of anhydrous conditions and activated molecular sieves to remove the water formed in the reaction was required. Even with these extra measures, the yields obtained were low.

β-lactam synthesis was primarily carried out using the Staudinger reaction, known since 1907. It is a cycloaddition reaction between a ketene and an imine under basic conditions. The ketene is generated from an acid chloride. The stereochemistry of the product varies depending on numerous factors, including the reaction conditions, the order of addition of the reagents and the substituents present on both the imine and on the acid chloride. A typical synthesis of a *trans* substituted compound according to the present invention by a Staudinger reaction is illustrated in Scheme 2.

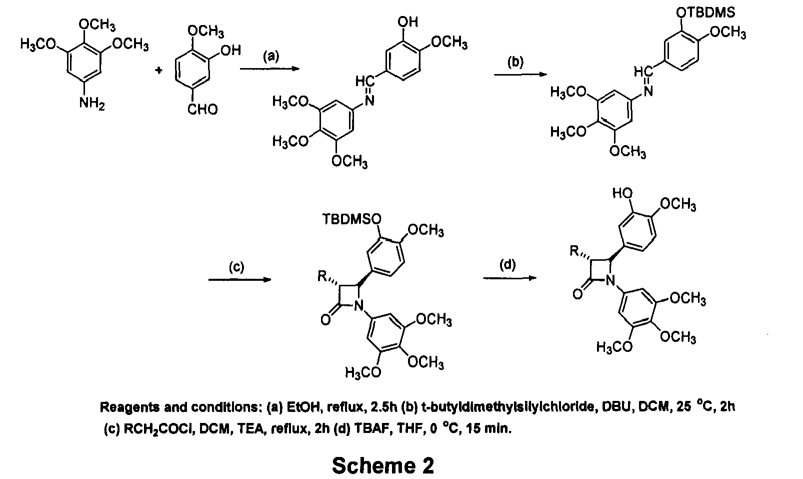

The appropriate imine (5 mmol) and triethylamine (15 mmol) were added to dry CH₂Cl₂ (50 mL) and the mixture brought to reflux at 60 °C. Once refluxing, the appropriate acetyl chloride (10 mmol) was injected dropwise through a rubber stopper. This mixture was left refluxing for 3 hours. The mixture was washed firstly with distilled water (50 mL) (twice) and then with saturated aqueous sodium bicarbonate solution (50 mL). The organic layer was dried by filtration through anhydrous sodium sulfate. The organic layer containing the product was collected and reduced *in vacuo.*

In certain instances the acid chloride was not commercially available. In these cases, one of two approaches was used (see Scheme 3). The first involves generation of the acid chloride from the corresponding acetic acid using thionyl chloride. The appropriate phenylacetic acid (10 mmol) was brought to reflux with thionyl chloride (12 mmol) in chloroform (30 mL). The chlorination reactions were monitored by IR until absorption appeared in the spectrum between v1790 cm⁻¹ and *v*1815 cm⁻¹. This peak is due to the -C=O stretching vibration in the acid chloride molecule. The solvent was evaporated and the acid chloride was used without further purification.

Secondly, the β-lactam could be formed directly from the phenylacetic acid using an acid-activating agent in a one-step reaction. Many acid-activating agents are known in literature, e.g. Mukaiyama's reagent (2-chloro-N-methylpyridinium iodide), ethyl chloroformate, trifluoroacetic anhydride, p-toluene-sulfonyl chloride and various phosphorous derived reagents. Triphosgene, or bis(trichloromethyl)carbonate, was used in our synthesis.

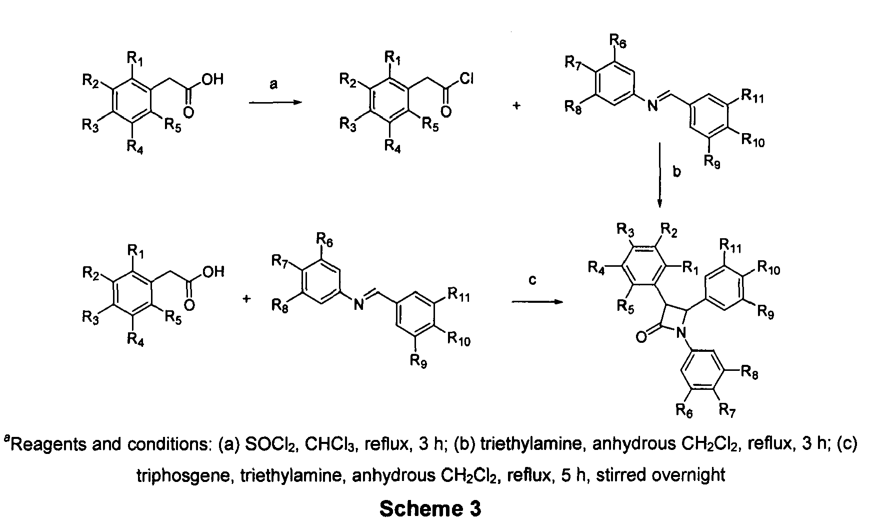

In selected cases, the Reformatsky reaction was used (see Scheme 4). It is a useful method for forming carbon-carbon bonds. The precursor is an organozinc-type compound. The Reformatsky reaction has the advantages of proceeding under neutral conditions, having a selective site of reaction, determined by position of halogen. It is limited by lower yields (when compared to the aldol reaction) and loss of control over the stereoselectivity of the products. Activated zinc gives a better yield. There are many ways to activate zinc, e.g. by washing with nitric acid). Various chemicals have been used for depassivating zinc, including 10% HCl and trimethylchlorosilane (TMCS). Previous work in our laboratory found TMCS to be better than both iodine and zinc washed with 10% nitric acid. Zinc pre-activated with trichloromethylsilane was used in microwave conditions. An investigation into the reaction with use of microwaves was carried out in our laboratory previously, with the result that the yield was slightly increased but, more importantly, the reaction time was decreased by 7.5 hours (30 minutes compared to 8 hours).

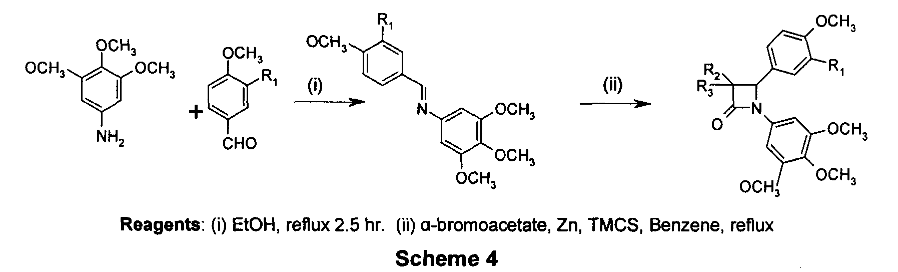

The antiproliferative activity of a number of compounds according to the present invention in MCF-7 breast cancer cell line are shown in Table 1. 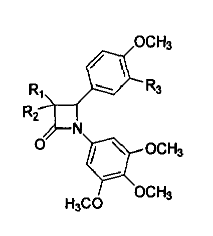 ^{a}all compounds tested were the *trans* isomers except for compounds 27and 28 which were both obtained as the *cis* isomers.

**Table 1 - antiproliferative activity in MCF-7 breast cancer cell line**

| **Compound Number**^{a} | **R₁** | **R₂** | **R₃** | **IC₅₀ (µM) MCF-7 cell line** |
|---|---|---|---|---|
| **1** | C₆H₅O | H | OH | 0.038 |
| **2** | CH₂=CH | H | OH | 0.0014 |
| **3** | (CH₃)CH=CH | H | OH | 0.019 |
| **4** | C₆H₅ | H | OH | 0.0096 |
| **5** | Cl | H | OH | 0.0175 |
| **6** | CH₃CH(OH) | H | OH | 0.0376 |
| **7** | C₆H₅CH=CHCH(OH) | H | OH | 0.0468 |
| **8** | C₆H₅ | H | H | 0.034 |
| **9** | 4-NH₂-C₆H₄ | H | H | 0.050 |
| **10** | 4-OH-C₆H₄ | H | H | 0.059 |
| **11** | 4-F-C₆H₄ | H | H | 0.042 |
| **12** | 2-Thienyl | H | H | 0.064 |
| **13** | C₆H₅ | H | NH₂ | 0.065 |
| **14** | 4-OH-C₆H₄ | H | OH | 0.0008 |
| **15** | 4-OH-C₆H₄ | H | NH₂ | 0.039 |
| **16** | 4-F-C₆H₄ | H | OH | 0.065 |
| **17** | 4-F-C₆H₄ | H | NH₂ | 0.055 |
| **18** | 2-Thienyl | H | OH | 0.0070 |
| **19** | 2-Thienyl | H | NH₂ | 0.041 |
| **20** | 3-Thienyl | H | OH | 0.010 |
| **21** | 3-Thienyl | H | H | 0.060 |
| **22** | 3-OH- C₆H₄ | H | H | 0.0689 |
| **23** | 4-NH₂- C₆H₄ | H | OH | 0.004 |
| **24** | 4-NH₂- C₆H₄ | H | NH₂ | 0.030 |
| **25** | H | H | OH | 0.017 |
| **26** | H | H | H | 0.039 |
| **27** | CH₃ | H | H | 0.047 |
| **28** | CH₃ | H | OH | 0.010 |

### Compound Characterisation Data

### All compounds are >98% pure by HPLC (mobile phase: acetonitrile: water [70:30])

***4-(3-Hydroxy-4-methoxy-phenyl)-3 phenoxy-1-(3,4,5-trimethoxy-phenyl)-azetidin-2-one (1)*** To a stirring, refluxing solution of the protected imine *[3-(tert-Butyldimethylsilanyloxy)-(4-methoxybenzylidene)-(3,4,5-trimethoxy phenyl)-amine* (5mmol) and triethylamine (6mmol) in anhydrous dichloromethane (40mL), a solution of the appropiate acid chloride (6mmol) in anhydrous dichloromethane (10mL) was added over 45 minutes under nitrogen. The reaction was kept at reflux during the day (8 hours) and at room temperature overnight (16 hours), continuously under nitrogen, until the starting material had disappeared as monitored by TLC in 50:50 hexane:ethyl acetate (total reaction time of 48-96 hours). A characteristic darkening of the reaction over time was observed. The reaction was transferred to a separating funnel and washed with water (2 x 100mL), with the organic layer being retained each time. The reaction was dried over Na₂SO₄ before the solvent was removed under reduced pressure and the silyl ether β-lactam isolated by flash chromatography over silica gel eluted with 1:1 *n*-hexane:ethyl acetate. The first silyl ether was characterised fully and subsequent silyl ether β-lactams were directly deprotected to yield the free phenols as follows. To a stirring solution of the sily ether β-lactam (2mmol) under N₂ and at 0°C in dry THF was added dropwise 1.0 M t-BAF solution in hexanes (2mL, 2mmol). The resulting solution was left to stir at 0 °C until reaction was complete as seen on TLC. Reaction was diluted with ethyl acetate (75mL) and washed with 0.1 M HCL_{aq} (100mL). The aqueous layer was further extracted with ethyl acetate (2x25mL). All the organic layers were collected and washed with H₂O (100mL), and saturated brine (100mL) before being dried over Na₂SO₄. Solvent was removed under reduced pressure to yield the free phenol. *4-[3-(t-Butyl-dimethyl-silanyloxy)-4-methoxy-phenyl]-3-phenoxy-1-(3,4,5-trimethoxyphenyl)-azetidin-2-one ;* Yield: *Cis* 61%, 1.73g, white resin *Trans* 21%, 0.59g white resin; IR (KBr v max): 1729 cm⁻¹(C=O); MP: Resin; ¹H: *Cis isomer* δ0.04 (6H, s, -tBDMSi), δ0.91 (9H, s, - tBDMSi), δ3.72 (3H, s, -OMe), δ3.74 (6H, s, -OMe), δ3.91 (3H, s, -OMe), δ5.28 (1H, d J=4.52 Hz, H₄), δ5.52 (1 H, d, J=5Hz, H₃), δ6.64-6.89 (10H, m, (ϕ-H); *Trans isomer* δ0.02 (6H, s, - tBDMSi), δ1.02 (9H, s, -tBDMSi), δ3.75 (3H, s, -OMe), δ3.77 (6H, s, -OMe), δ3.92 (3H, s, - OMe), δ4.89 (1 H, d J=1.52Hz, H₄), δ5.10 (1 H, d J=1.5Hz, H₃), δ6.58-6.87 (10H, m, ϕ-H); ¹³C NMR (100 MHz, CDCl₃): *Cis isomer* δ-4.20, δ-4.16, δ17.88, δ23.48 (OtBDMS) δ55.43, δ55.73, δ60.59 (OMe), δ61.89 (C₄), δ81.06 (C₃), δ94.87 (C₂'&C₆'), δ113.59(C5"'), δ114.51 (C₂"&C₆"), δ117.44 (C₂"'), δ119.41 (C₄"), δ120.27 (C₆"'), δ125.12 (C₃" C₅"), δ128.02 (C₄'), δ132.39 (C₁'), δ137.83 (C₁"'), δ142.02 (C₃"'), δ148.27(C₃'C₅'), δ151.15 (C₄"'), δ156.69 (C₁"), δ162.67 (C₂); *Trans isomer* δ-4.22, δ-4.18, δ17.56, δ24.09, δ54.82, δ54.95, δ59.22 (OMe), δ62.32 (C₄), δ82.56 (C₃), δ95.17 (C₂'&C₆'), δ111.38 (C₂"&C₆"), δ114.18 (C₅"'), δ117.58 (Cz"'), δ119.79 (C₄"), δ120.39 (C₆"'), δ128.22 (C₄'), δ131.35 (C₃"&C₅"), δ132.29 (C₁'), δ138.28 (C₁'"). δ143.11 (C₃"'), δ149.67 (C₄"'), δ149.99 (C₃'&C₅'), δ157.32 (C₁"), δ161.07 (C₂); **HRMS**: C₃1H₃₉NO₇Si M⁺+Na requires 588.2393, found 588.2402 (1.5ppm).

***4-(3-Hydroxy-4-methoxy-phenyl)-3-phenoxy-1-(3,4,5-trimethoxy-phenyl)-azetidin-2-one (1)*** Yield: 73%, 1.65g; MP: 176-180 °C; IR (KBr max): 1739 cm⁻¹ (C=O), 3401 cm⁻¹ (-OH) 1737 cm⁻¹(C=O), 3498 cm⁻¹(OH); ¹H NMR (400MHz, CDCl₃): *Trans* δ3.65 (s, 3H, OMe), δ3.70 (s, 6H, OMe), δ3.88 (s, 3H, OMe), δ5.10 (d, J=1.52Hz, 1H, H₄), δ5.35 (d, J=1.52, 1H, H₃), δ6.70 (s, 2H, C₂'&C₆'), δ6.92-7.31 (m, 8H, Ar-H), δ7.87 (s, 1H, OH); *Cis* δ3.74 (s, 6H, OMe), δ3.79 (s, 3H, OMe), δ3.86 (s, 3H, OMe), δ5.29 (d, J=5.0Hz, 1H, H₄), δ5.53 (d, J=5.0Hz, 1 H, H₃), δ6.65 (s, 2H, H₂'&H₆'), δ6.78-7.21 (m, 8H, H₂" H₃" H₄" H₅" H₆" H₂"' H₅"' H₆"') ¹³C: *Cis isomer* δ55.40, δ55.64, δ60.50 (OMe), δ61.64 (C₄), δ80.72 (C₃), δ94.84 (C₂'&C₆'), δ109.94 (C₂"&C₆"), δ113.90 (C₂"'), δ115.34 (C₅"'), δ119.68 (C4"), δ121.75 (C₆"'), δ125.12 (C4'), δ128_{.}84 (C₃"&C₅"), δ132_{.}67 (C₁'), δ145.10 (C₁'), δ145.12 (C₁'"). δ146.42 (C₄"'), δ153.05 (C₃'&C₅'), δ156.64 (C₁"), δ162.54 (C₂); *Trans isomer* δ54.88, δ54.94, δ59.21 (OMe), δ63.01 (C₄), δ86.47 (C₃), δ95.15 (C₂'&C₆'), δ111.31 (C₂"&C₆"), δ112.93 (C₂"'), δ114.90 (C₅"'), δ118.29 (C₄"), δ121.61 (C₆"'), δ128.23 (C₄'), δ129.13 (C₃"&C₅"), δ132.73 (C₁'), δ134.23 (C₁"'), δ146.81 (C₃"'), δ147.74 (C₄"'), δ153.26 (C₃'&C₅'), δ156.85 (C₁"), δ161.61 (C₂); HRMS: C₂₅H₂₅NO₇; *Cis* isomer M⁺+Na requires (m/e) 474.1529, found (m/e) 474.1529 (0 ppm) *Trans* isomer M⁺+Na requires 474.1526, found 474.1533 (0.9ppm)

***4-(3-Hydroxy 4-methoxy-phenyl)-1-(3,4,5-trimethoxy-phenyl)-3-vinyl-azetidin-2-one* (2)** To the silyl ether imine *(3-(tert-Butyldimethylsilanyloxy)-(4-methoxybenzylidene)-(3,4,5-trimethoxy-phenyl)-amine* (5mmol) in DCM (25mL), and (TEA 6mmol) stirring at reflux under N₂ was added a solution of the appropriate acid chloride (6mmol) in DCM (10mL) dropwise over the course of 25 minutes. The reaction was then left to reflux under N₂ for 6 hours and at room temperatures overnight. The reaction was diluted with DCM and washed with H₂O (2x100mL). The organic layer was collected and dried over MgSO₄ before the solvent was removed under reduced pressure. The deprotection of the β-lactam product was carried out by treatment with tBAF in dry THF at 0°C under N₂ as previously described. Yield: 18%, 347mg, brown oil; MP: oil; IR (NaCl ^{v} max): 1732 cm⁻¹(C=O). 3408 cm⁻¹(OH); ¹H NMR (400MHz, CDCl₃: δ3.75 (m, 7H, OMe & H₃), δ3.78 (s, 3H, OMe), δ3.91 (s, 3H, OMe), δ4.69 (d, J=2.04Hz, 1 H, H₄), δ5.34 (t, J=8Hz,10.56, 2H, CHCH₂), δ5.78 (s, 1 H, OH), δ5.98-6.38 (m, 1H, CHCH₂), δ6.57-6.94 (m, 5H, H₂' H₆' H₂" H₅" H₆"); ¹³C NMR (100 MHz, CDCl₃): δ55.56,655.59,660.86 (OMe), δ60.50 (C₃), δ63.37 (C₄), δ94.27 (C₂'&C₆'), δ110.51 (C₁"), δ111.54 (C₂"), δ111.57 (C₅"), δ117.31 (C₆"), δ119.42 (CHCH₂), δ129.91 (C₄'), δ130.09 (CHCH₂), δ133.38 (C₁'), δ134.12 (C₁"), δ145.84 (C₃"), δ146.37 (C₄"), δ153.01 (C₃'&C₅'), δ164.85 (C₂); HRMS M⁺Na requires 408.1423, found 408.1420 (-0.8ppm)

***4-(3-Hydroxy-4-methoxy-phenyl)-3-isopropenyl-1-(3,4,5-trimethoxy-phenyl)-azetidin-2-one* (3)** Yield: 22%, 439mg, brown oil; MP: oil; IR (NaCI v max): 1717 cm⁻¹(C=O), 3532 cm⁻¹(OH); ¹H NMR (400MHz, CDCl₃): δ1.84 (s, 3H, CH₃), δ3.72 (d, J=1.78, 1H, H₃), δ3.74 (s, 6H, OMe), δ3.78 (s, 3H, OMe), δ3.91 (s, 3H, OMe), δ4.72 (d, J=1.78, 1 H, H₄), δ5.01 (d, J=14.3Hz, 1H, CH₂), δ5.08 (d, J=14.38Hz, 1H, CH₂, δ5.79 (br, s, 1H, OH), δ6.59 (s, 2H, H₂'&H₆'), δ6.86-6.90 (m, 3H, H₂" H₅" H₆") ; ¹³C NMR (100 MHz, CDCl₃): δ20.10 (C₇), δ55.86, δ55.90, δ60.22 (OMe), δ60.76 (C₃), δ66.68 (C₄), δ94.70 (C₂'&C₆'), δ110.90 (C₂"), δ111.98 (C₅"), δ114.15 (C₆), δ117.58 (C₆"), δ130.67 (C₄'), δ133.63 (C₁'&C₁"), δ134.35 (C₂"), δ138.03 (C₃"), δ146.19 (C₅), δ146.67 (C₄"),δ6153.35 (C₃'&C₅'), δ165.04 (C₂); HRMS: M⁺+Na requires 422.1580, found 422.1596 (3.9ppm)

***4-(3-Hydroxy-4-methoxyphenyl)-3-phenyl-1-(3,4,5-trimethoxyphenyl)-azetidin-2-one (4)*** was obtained as an off white solid material.; Melting point: 110°C; IR: KBr disk u: 1718.23 cm⁻¹ (C=O, β-lactam); ¹H NMR (400 MHz, CDCl₃) δ3.73 (s, 6H), δ 3.78 (s, 3H), 6 3.91 (s, 3H), δ 4.27 (d, 1 H, J=2.52 Hz), δ 4.81 (d, 1H, J=2.48 Hz), δ 5.75 (s, 1 H), δ 6.63 (s, 2H), 6 6.86 - 6.93 (m, 2H), δ 7.00 (d, 1 H, J=2 Hz), δ 7.31 - 7.39 (m, 5H); ¹³C NMR (400 MHz, CDCl₃) δ 55.58, δ 55.60, δ 60.51, δ 63.36, δ 64.49, δ 94.42, δ 110.59, δ 111.56, δ 117.40, δ 126.97, δ 127.43, 6 128.57, δ 130.10, δ 133.27, δ 134.02, δ 134.31, δ 145.91, δ 146.43, δ 153.05, δ 165.14; EIMS (HR): C₂₅H₂₅NO₆Na; Mass (+Na) 458.1575; calculated mass (+Na) 458.1580; error -1.0 ppm

***3-Chloro-4-(3-hydroxy-4-methoxy-phenyl)-1-(3,4,5-trimethoxy-phenyl)-azetidin-2-one (5)*** Yield: 34%, 668mg, brown oil; MP: oil; IR (NaCl ^{v} max): 1770 cm⁻¹(C=O), 3417 cm^{- 1}(OH); ¹H NMR (400MHz, CDCl₃): 63.74 (s, 6H OMe), 63.78 (s, 3H OMe), 63.92 (s, 3H OMe), δ4.61 (d, J=2.04Hz 1 H H₃), 64.89 (d, J=1.52Hz 1 H H₃), 65.81 (s, 1 H OH), δ6.56 (s, 2H H₂'H₆'), 66.87-6.95 (m, 3H H₂" H₅" H₆"); ¹³C NMR (100 MHz, CDCl₃): δ55.59, δ55.63, δ60.51 (OMe), δ62.67 (C₃), δ65.63 (C₄), δ94.81 (C₂'&C₆'), δ110.61 (C₂"), δ111.58 (C₅"), δ117.72 (C₆"), δ127.52 (C₄'), δ132.50 (C₁'), δ134.63 (C₁"), δ146.04 (C₃"), δ147.00 (C₄"), δ153.08 (C₃'&C₅'), δ160.22 (C₂); HRMS: M⁺Na calculated 416.0877, found 416.0897, (4.8ppm)

***3-(1-Hydroxy ethyl)-4-(3-hydroxy-4-methoxy-phenyl)-1-(3,4,5-trimethoxy-phenyl)-azetidin-2-one* (6)** To a solution of *4-[3-(tert-Butyldimethylsilanyloxy)-4-methoxyphenyl]-1-(3,4,5-trimethoxyphenyl)-azetidin-2-one* (125mg, 0.264mmol) in dry THF (3mL) under N₂ at - 78°C (dry ice and acetone) was added 2.0M LDA solution (0.264mL, 0.528mmol). The resulting solution was left to stir for 5 minutes before a solution of the appropriately substituted aldehyde (49mg, 0.396mmol) in dry THF (1.5mL) was added. The reaction was left to stir for 30 minutes at -78°C, then poured onto NaClₛₐₜ (25mL). The resulting solution was extracted with ethyl acetate (50mL), and the solvent was dried over Na₂SO₄ before being removed under reduced pressure. Gross impurities were removed from the resulting residue by passage through a short pad (5cm) of silica with DCM to yield addition product. To a stirring solution of the addition product (2mmol) in dry THF (10mL) was added a solution of 1.0M tBAF in hexanes (2mL, 2mmol) under N₂ at 0°C. The reaction was stirred for a further 90 minutes. Reaction was diluted with ethyl acetate (75mL) and washed with 0.1M HCLₐ (100mL). The aqueous layer was further extracted with ethyl acetate (2x25mL). All the organic layers were collected and washed with H₂O (100mL), and saturated brine (100mL) before being dried over Na₂SO₄ and solvent was removed under reduced pressure. Purification was carried out by chromatography using a Biotage™ SP1 chromatography system using a +12M column and detection set at 280nM and a fraction volume of 12mL. A gradient elution of 2% ethyl acetate in *n*-hexane to 100% ethyl acetate over 15 column volumes was used. Yield: 17%, 36mg, brown oil; MP: Oil; IR (NaCl v max): 1738 cm⁻¹(C=O), 3427 cm⁻¹(OH); ¹H NMR (400MHz, CDCl₃): δ1.33 (d, J=6.28Hz, 1H, CH₃), δ1.40 (d, J=6.52Hz, 2H, CH₃). δ2.58 (br s, 1H, OH), δ3.14 (m, 1H, H₃), δ3.72 (s, 6H, OMe), δ3.76 (s, 3H, OMe), δ3.89 (s, 3H, OMe), δ4.24 (q, J=6.04Hz, 4.24Hz, 0.66H, H₅), δ4.36 (q, J=5.76Hz, 4.01 Hz, 0.33H, H₅), δ4.77 (d, J=2.28Hz, 0.6H,H₄), δ4.99 (d, J=2.28, 0.4H, H₄), δ5.95 (s, 0.6H, OH), δ5.96 (s, 0.4H, OH), δ6.54 (s, 2H, H₂'&H₆'), δ6.83-7.01 (m, 3H, H₂" H₅" H₆"); ¹³C NMR (100 MHz, CDCl₃): δ21.34, δ21.52 (CH₃), δ55.99, δ56.06 (C₄), δ57.64 δ57.68 (C₃), δ56.68, δ60.93, δ64.94 (OMe), δ66.05 δ66.10 (C₅), δ94.70, δ94.75 (C₂'&C₆'), δ111.05 (C₅"), δ112.16, δ112.22 (C₂"), δ117.88 (C₆"), δ130.50 (C₄'), δ130.97 (C₁'), δ133.67, δ134.32 (C₁"), δ146.25,δ146.32 (C₃"), δ146.88 (C₃'&C₅'), δ153.43 (C₄"), δ165.89,δ166.06 (C₂); HRMS: M⁺+Na calculated 426.1529, found 426.1540 2.6ppm.

***4-(3-Hydroxy-4-methoxy-phenyl)-3-(1-hydroxy-3-phenyl-allyl)-1-(3,4,5-trimethoxyphenyl)-azetidin-2-one (7)*** The procedure was carried out as above. Yield: 38%, 99mg, brown oil; ; IR (NaCl v max): 1732cm⁻¹ (C=O) 3418 cm⁻¹(OH); ¹H NMR (400MHz, CDCl₃): δ3.21 (br, s, 1H, OH), δ3.34 (dd, J=2.55 5.63Hz, 0.53H, H₃), δ3.37 (dd, J=2.55, 5.62 Hz, 0.57H, H₃), δ3.69 (s, 6H, OMe), δ3.76 (s, 3H, OMe), δ3.85 (s, 3H, OMe), δ4.75 (t, J=6.13Hz, 12.40Hz, 0.43H, H₅), δ4.86 (d, J=2.45Hz, 0.43H, H₄), δ4.91 (t, J=3.82 7.63Hz, 0.57H, H₅), δ5.03 (d, J=2.45Hz, 0.53H, H₄), δ5.81-5.98 (m, 1 H, H₆), δ6.24 (dd, J=5.54, 16.28Hz, 0.57H, H₇), δ6.41 (dd, J=5.54, 16.28, 0.43H, H₇), δ6.58 (s, 2H, H₂' H₆'), δ6.72-6.96 (m, 3H, H₂"' H₅"' H₆"'), δ7.35-7.39 (m, 5H, H₂" H₃" H₄" H₅" H₆") ; ¹³C NMR (100 MHz, CDCl₃): δ53.32, δ55.84, δ56.17, δ60.75 (OMe) δ57.35, δ57.41 (C₄) δ64.81, δ68.41 (C₃) δ70.77 (C₅) δ94.81 (C₃'C₅') δ94.86, δ95.12, δ110.61 (C₅"') δ110.95, δ110.98 (C₂"') δ117.84, δ117.91 (C₆"') δ126.37, δ126.61 (C₆) δ128.03, δ128.46 (C₇) δ129.49 (C₄') δ130.33 (C₂"C₆") δ131.45 (C₄') δ133.12 (C₁') δ134.48 (C₁") δ136.20 (C₄') δ146.16 (C₃" C₅") δ146.61 (C₄"') δ148.29 (C₃"') δ153.29,δ153.31 (C₁"') δ165.48, δ165.48 (C₂); HRMS: C₂₈H₂₉NO₇ M⁺+Na requires 514.1842, found 514.1826 (-3.1ppm).

**4-(4-Methoxy-phenyl)-3-phenyl-1-(3,4,5-trimethoxy-phenyl)-azetidin-2-one (8)** was obtained as a white crystalline solid (6.77% yield).Melting point: 108°C; IR: NaCl u: 1753.27 cm^{- 1} (C=O, β-actam); ¹H NMR (400 MHz, CDCl₃) δ 3.72 (s, 6H, -OCH₃), δ 3.77 (s, 3H, -OCH₃), δ 3.83 (s, 3H, -OCH₃), δ 4.29 (d, 1H, J=2.52 Hz), δ 4.87 (d, 1H, J=2.52 Hz), δ 6.60 (s, 2H), δ 6.95 (d, 2H, J=8.52 Hz), δ 7.26 (s, 1 H), δ 7.32 - 7.40 (m, 7H); ¹³C NMR (400 MHz, CDCl₃) δ 54.92, δ 55.57, δ 60.52, δ 63.40, δ 64.59, δ 94.38, δ 114.24, δ 126.89, δ 127.00, δ 127.45, δ 128.59, δ 128.85, δ 133.28, δ 134.00, δ 134.33, δ 153.05, δ 159.49, δ 165.21; EIMS (HR): C₂₅H₂₅NO₅Na; Mass (+Na) 442.1631; calculated mass (+Na) 442.1630; error +0.1 ppm.

**3-(4-Amino-phenyl)-4-(4-methoxy-phenyl)-1-(3,4,5-trimethoxyphenyl)-azetidin-2-**one (9) was prepared from 4-(4-Methoxy-phenyl)-3-(4-nitro-phenyl)-1-(3,4,5-trimethoxy-phenyl)-azetidin-2-one. It was a brown oil and was obtained in 41.8% yield. IR: NaCl disk u: 1742.02 cm⁻¹ (C=O, β-lactam); ¹H NMR (400 MHz, CDCl₃) δ 3.74-3.84 (t, 12H), δ4.19 (d, 1H, J=2 Hz), δ 4.80 (d, 1H, J=2.48 Hz), δ 6.62 (s, 2H), δ 6.73 (d, 1H, J=8.04 Hz), δ 6.95 (d, 2H, J=9.04 Hz), δ 7.13 (d, 1H. J=8.04 Hz), δ 7.29 (s, 2H), δ 7.35 (d, 2H, J=8.56 Hz); ¹³C NMR (400 MHz, CDCl₃) δ 54.91, δ 55.56, δ 60.51, δ 63.89, δ 64.28, δ 94.37, δ 114.16, δ 115.23, δ 124.35, δ 126.83, δ 128.04, δ 129.08, δ 133.39, δ 133.91, δ 153.03, δ159.39, δ 165.97; EIMS (HR): C₂₅H₂₆N₂O₅; Mass 435.1916; calculated mass 435.1920; error -0.9 ppm.

**3-(4-Hydroxy-phenyl)-4-(4-methoxy-phenyl)-1-(3,4,5-trimethoxy-phenyl)-azetidin-2-**one (10) was formed as a white crystalline powder (100% yield). Melting point: 155°C; IR: KBr disk u: 1725.19 cm⁻¹ (C=O, β-lactam); ¹H NMR (400 MHz, CDCl₃) δ 3.72 (s, 6H), δ 3.78 (s, 3H), δ 3.82 (s, 3H), δ 4.18 (s, 1H), δ 4.84 (s, 1H), δ 6.26 (s, 1 H), δ 6.61 (s, 2H), δ 6.73 (d, 2H, J=7.6 Hz), δ 6.93 (d, 2H, J=7.6 Hz), δ 7.09 (d, 2H, J=8.2 Hz), δ 7.32 (d, 2H, J=8.16 Hz); ¹³C NMR (400 MHz, CDCl₃) δ 54.92, δ 55.58, δ 60.53, δ 63.93, δ 64.03, δ 94.53, δ 114.21, δ 115.54, δ 125.44, δ 126.88, δ 128.26, δ 128.66, δ 133.11, δ 134.09, δ 153.06, δ 155.38, δ 159.48, δ 166.56; EIMS (HR): C₂₅H₂₅NO₆Na; mass (+Na) 458.1595; calculated mass (+Na) 458.1580; error +3.4 ppm.

**3-(4-Fluoro-phenyl)-4-(4-methoxy-phenyl)-1-(3,4,5-trimethoxy-phenyl)-azetidin-2-one (11)** was obtained as a white crystalline material (7.46% yield).Melting point: 120°C; IR: NaCl u: 1743.68cm⁻¹ (C=O, β-lactam); ¹H NMR (400 MHz, CDCl₃) δ 3.72 (s, 6H), δ 3.77 (s, 3H), δ 3.83 (s, 3H), δ 4.26 (d, 1H, J=2.44 Hz), δ 4.82 (d, 1 H, J=1.92 Hz), δ 6.59 (s, 2H), δ 6.95 (d, 2H, J=8.8 Hz), δ 7.04 - 7.09 (t, 2H), 7.27 - 7.32 (m, 4H); ¹³C NMR (400 MHz, CDCl₃) δ 54.93, δ 55.57, δ 60.51, δ 63.53, δ 63.79, δ 94.40, δ 114.29, δ 115.43, δ 115.64, δ 126.66, δ 126.85, δ 128.60, δ 128.68, δ 130.10, δ 130.13, δ 133.16, δ 134.08, δ 153.07, δ 159.57, δ 160.70, δ 163.16, δ 165.00; EIMS (HR): C₂₅H₂₄NO₅NaF; mass (+Na) 460.1542; calculated mass (+Na) 460.1536; error +1.3 ppm.

**4-(4-Methoxyphenyl)-3-thiophen-2-yl-1-(3,4,5-trimethoxyphenyl)-azetidin-2-one (12)** was obtained as a white powder (4.55% yield).Melting point: 115°C; IR: NaCl u: 1756.78cm⁻¹ (C=O, β-lactam); ¹H NMR (400 MHz, CDCl₃) δ 3.72 (s, 6H), δ 3.77 (s, 3H), δ 3.82 (s, 3H), δ 4.47 (d, 1H. J=2 Hz), δ 4.90 (d, 1H, J=2.52 Hz), δ 6.59 (s, 2H), δ 6.95 (d, 2H, J=8.56 Hz), δ 7.01 - 7.03 (t, 1H). δ 7.08 (d, 1H, J=3.48 Hz), δ 7.26 (d, 1H); ¹³C NMR (400 MHz, CDCl₃) δ 54.93, δ 55.58, δ 59.78, δ 60.52, δ 64.12, δ 94.46, δ 113.86, δ 114.27, δ 124.43, δ 124.87, δ 125.29, δ 126.82, δ 126.90, δ 128.29, δ 133.19, δ 134.11, δ 135.70, δ 148.98, δ 153.06, δ 159.60, δ 163.98; EIMS (HR): C₂₃H₂₃NO₅NaS; mass (+Na) 448.1186; calculated mass (+Na) 448.1195; error -1.9 ppm.

**4-(3-Amino-4-methoxy-phenyl)-3-phenyl-1-(3,4,5-trimethoxy-phenyl)-azetidin-2-one (13)** was prepared from 4-(4-Methoxy-3-nitro-phenyl)-3-phenyl-1-(3,4,5-trimethoxy-phenyl)-azetidin-2-one. It was a yellow oil and was obtained in 1.93% yield. IR: NaCl disk u: 1742.61 cm⁻¹ (C=O, β-lactam); ¹H NMR (400 MHz, CDCl₃) δ 3.76 (s, 6H, -OCH₃), δ 3.80 (s, 3H, -OCH₃), δ 3.89 (s, 3H, -OCH₃), δ 4.30 (d, 1H. J=2.52 Hz, Hₓ), δ 4.79 (d, 1H, J=2.52 Hz, Hₓ), δ 6.66 (s, 2H, Ar-H), δ 2.84 (d, 3H, J=4.48 Hz, Ar-H), δ 7.33 - 7.39 (m, 5H, Ar-H); ¹³C NMR (400 MHz, CDCl₃) δ 53.00, δ 55.13, δ 55.59, δ 60.51, δ 63.64, δ 64.46, δ 94.39, δ 110.05, δ 111.20, δ 116.04, δ127.00, δ127.37, δ128.54, δ129.47, δ133.42, δ133.94, δ134.49, δ136.41, δ 147.21, δ153.02, δ165.34; EIMS (HR): C₂₅H₂₆N₂O₅; Mass 435.1913; calculated mass 435.1920; error -1.6 ppm.

***4-(3-Hydroxy 4-methoxy-phenyl)-3-(4-hydroxy-phenyl)-1-(3,4,5-trimethoxy-phenyl)-azetidin-2-one (14)*** was formed as a white powder (2.87% yield).Melting point: 152°C; IR: NaCl disk u: 1720.58 cm-¹ (C=O, β-lactam); ¹H NMR (400 MHz, CDCl₃) δ 3.65 (s, 3H), δ 3.70 (s, 6H), δ 3.86 (s, 3H), δ 4.26 (d, 1H. J=2.44 Hz), δ 4.98 (d, 1H, J=2.44 Hz), δ 6.71 (s, 2H), δ 6.87 (d, 2H, J=8.8 Hz), δ.00 (s, 3H), δ.22 (d, 2H, J=8.8 Hz); ¹³ C NMR (400 MHz, CDCl₃) δ54.93, δ 59.23, δ 63.05, δ63.76, δ94.66, δ11.31, δ12.22, δ15.13, δ17.48, δ25.72, δ28.25, δ 130.39, δ133.49, δ134.12, δ146.70, δ147.32, δ153.25, δ156.50, δ165.27; EIMS (HR): C₂₅H₂₅NO₇Na; mass (+Na) 474.1548; calculated mass (+Na) 474.1529; error + 4.1 ppm.

***4-(3-Amino-4-methoxy-phenyl)-3-(4-hydroxy-phenyl)-1-(3,4,5-trimethoxy-phenyl)-azetidin-2-one*** (15) was formed as a pink powder.; Melting point: 177°C; EIMS (HR): C₂₅H₂₆N₂O₆Na; mass (+Na) 473.1699; calculated mass (+Na) 473.1689; error + 2.2 ppm.

***3-(4-Fluoro-phenyl)-4-(3-hydroxy-4-methoxy-phenyl)-1-(3,4,5-trimethoxy-phenyl)-azetidin-2-one** (16)* was formed as yellow oil (2.06 % yield).IR: KBr disk u: 1744.43 cm-¹ (C=O, β-lactam); ¹H NMR (400 MHz, CDCl₃) δ 3.76 (s, 6H), δ3.80 (s, 3H), δ3.93 (s, 3H), δ4.27 (d, 1H, J=2 Hz), δ4.79 (d, 1H. J=2.48 Hz), δ5.78 (s, 1H). δ6.63 (s, 2H), δ6.91 - 6.93 (m, 2H), δ 7.00 (s, 1 H), δ7.06 - 7.11 (t, 2H), δ7.29 - 7.34 (m, 2H); ¹³ C NMR (400 MHz, CDCl₃) δ55.61, 6 60.51, δ63.50, δ63.70, δ94.44, δ110.62, δ111.49, δ 115.41, δ115.62, δ117.36, δ128.59, δ 128.68, δ129.86, δ130.10, δ133.15, δ 134.11, δ145.96, δ146.51, δ 153.07, δ160.69, δ 163.15, δ164.90; ElMS (HR): C₂₅H₂₄NO₆FNa; mass (+Na) 476.1484; calculated mass (+Na) 476.1485; error - 0.3ppm.

***4-(3-Amino-4-methoxy-phenyl)-3-(4-fluoro-phenyl)-1-(3,4,5-trimethoxy-phenyl)-azetidin-2-one*** (17) was prepared from 3-(4-Fluoro-phenyl)-4-(4-methoxy-3-nitro-phenyl)-1-(3,4,5-trimethoxy-phenyl)-azetidin-2-one (38) and was obtained as a brown residue (59.5% yield).lR: NaCl disk u: 1739.19 cm-¹ (C=O, β-lactam); ¹H NMR (400 MHz, CDCl₃) δ3.75 (s, 6H), δ3.80 (s, 3H), δ3.88 (s, 3H), δ4.27 (d, 1 H, J=2.04 Hz), δ4.74 (d, 1H, J=2.48 Hz), δ6.65 (s, 2H), δ6.77 - 6.80 (m, 3H), δ7.05 - 7.10 (t, 2H), δ7.29 - 7.33 (m, 2H); ¹³ C NMR (400 MHz, CDCl₃) δ55.13, δ55.59, δ60.51, δ63.64, δ63.79, δ74.37, δ94.40, δ110.03, δ111.00, δ 115.36, δ 115.57, δ115.90, δ128.60, δ128.68, δ129.21, δ130.26, δ130.29, δ133.33, δ 134.02, δ136.64, δ147.23, δ153.04, δ160.65, δ163.11, δ165.14; ElMS (HR): C₂₅H₂₅N₂O₅NaF; Mass (+Na) 475.1653; calculated mass +475.1645; error - 2.1 ppm.

***4-(3-Hydroxy-4-methoxy-phenyl)-3-thiophen-2-yl-1-(3,4,5-trimethoxy-phenyl)-azetidin-2-one (18)*** was formed as brown crystals (1.3% yield).Melting point: 113-114°C; lR: KBr disk u: 1721.07cm⁻¹ (C=O, β-lactam); ¹H NMR (400 MHz, CDCl₃) δ3.76 (s, 6H), δ3.80 (s, 3H), δ3.94 (s, 3H), δ4.48 (d, 1H. J=2 Hz), δ4.87 (d, 1H, J=2.52 Hz), δ5.75 (s, 1 H), δ6.62 (s, 2H), δ6.89 - 6.95 (m, 2H), δ7.01 - 7.03 (m, 1 H), δ7.29 - 7.32 (m, 1 H); ¹³C NMR (400 MHz, CDCl₃) δ55.58, δ55.61, δ 59.70, δ60.51, δ64.07, δ94.50, δ110.60, δ111.46, δ117.36, δ 124.86, δ125.28, δ126.87, δ129.54, δ133.18, δ134.15, δ135.68, δ145.93, δ146.53, δ 149.32, δ153.06, δ163.90; ElMS (HR): C₂₃H₂₃NO₆SNa; mass (+Na) 464.1124; calculated mass (+Na) 464.1144; error - 4.3ppm.

***4-(3-Amino-4-methoxy-phenyl)-3-thiophen-2-yl-1-(3,4,5-trimethoxy-phenyl)-azetidin-2-one (19)*** was prepared from 4-(4-Methoxy-3-nitro-phenyl)-3-thiophen-2-yl-1-(3,4,5-trimethoxy-phenyl)-azetidin-2-one (39) and was obtained as a brown residue (48.47% yield).lR: NaCl disk u: 1749.94 cm⁻¹ (C=O, β-lactam); ¹H NMR (400 MHz, CDCl₃) δ3.76 (s, 6H), 6 3.80 (s, 3H), δ3.89 (s, 3H), δ4.49 (d, 1H, J=2 Hz), δ4.83 (d, 1H, J=2.52 Hz), δ6.64 (s, 2H), δ6.78 - 6.81 (m, 3H), δ7.03 - 7.05 (m, 1 H), δ7.08 - 7.09 (m, 1 H), δ7.29 - 7.31 (m, 1 H) ; ¹³ C NMR (400 MHz, CDCl₃) δ55.59, δ56.07, 6660.13, δ60.98, δ64.84, δ94.93, δ110.53, 6111.56, δ 116.43, δ125.25, δ125.70, δ127.31, δ129.34, δ133.81, δ134.50, δ136.36, δ136.96, δ 147.76, δ153.50, δ164.60.

***4-(3-Hydroxy-4-methoxy-phenyl)-3-thiophen-3-yl-1-(3,4,5-trimethoxy-phenyl)-azetidin-2-one (20)*** was formed as a pale pink solid (18.61% yield).; Melting point: 151 - 152°C; lR: KBr u: 1739.65 cm-¹ (C=O, β-lactam), 3187.91 cm-¹ (-OH); ¹H NMR (400 MHz, CDCl₃) δ3.76 (s, 6H, -OCH₃), 6 3.80 (s, 3H, -OCH₃), δ3.93 (s, 3H, -OCH₃), δ4.34 (d, 1H, J=2.24, Hₓ). δ4.82 (d, 1H, J=4.82, Hₓ), δ5.77 (s, 1H, -OH), δ6.63 (s, 2H, Ar-H), δ6.89 - 6.96 (m, 2H, Ar-H), δ7.02 (m, 1H, Ar-H), δ7.09 - 7.11 (m, 1 H, Ar-H), δ7.29 - 7.31 (m, 1 H, Ar-H), δ 7.39 - 7.41 (m, 1H, Ar-H); ¹³C NMR (400 MHz, CDCl₃) δ56.05 (-OCH₃), δ56.09 (-OCH₃), δ 60.48 (Cₓ). δ60.98 (-OCH₃), δ63.32 (Cₓ), δ94.87 (Ar-C), δ111.06 (Ar-C), δ111.98 (Ar-C), δ 117.81 (Ar-C), δ122.44 (Ar-C), δ,126.33 (Ar-C), δ126.85 (Ar-C), δ130.43 (Ar-C), δ133.78 (ArC), δ134.65 (Ar-C), δ146.39 (Ar-C), δ146.93 (Ar-C), δ153.53 (Ar-C), δ165.28 (-C=O); ElMS (HR): C₂₃H₂₃NO₆SNa mass (+Na) 464.1153; calculated mass (+Na) 464.1144; error +2.0 ppm.

***4-(4-Methoxy-phenyl)-3-thiophen-3-yl-1-(3,4,5-trimethoxy-phenyl)-azetidin-2-one*** (21) was formed as a off-white powder (19.23% yield).; Melting point: 130°C; lR: KBr u: 1750.82 cm-¹ (C=O, β-lactam); ¹H NMR (400 MHz, CDCl₃) 6 3.74 (s, 6H, -OCH₃), δ3.79 (s, 3H, -OCH₃), δ3.85 (s, 3H, -OCH₃), δ4.35 (d, 1 H, J=2.52 Hz, Hₓ), δ4.87 (d, 1 H, J=2.52 Hz, Hₓ), δ6.61 (s, 2H, Ar-H), δ6.96 - 6.98 (m, 2H, Ar-H), δ7.09 - 7.11 (m, 1 H, Ar-H), δ7.29 - 7.30 (m, 1 H, Ar-H), δ7.36 - 7.40 (m, 3H, Ar-H). ¹³C NMR (400 MHz, CDCl₃) 6 54.93 (-OCH₃), 6 55.56 (-OCH₃), δ 60.09 (-OCH₃), δ60.52 (C₃), δ62.89 (C₄), δ94.34 (Ar-C), δ114.25 (Ar-C), δ122.01 (Ar-C), δ 125.85 (Ar-C), δ126.42 (Ar-C), δ126.85 (Ar-C), δ133.33 (Ar-C), δ134.20, (Ar-C), δ153.05 (ArC), δ159.52 (Ar-C), δ164.89 (-C=O); ElMS (HR): C₂₃H₂₃NO₅NaS; mass (+Na) 448.1189; calculated mass (+Na) 448.1195; error -1.3 ppm.

***3-(3-Hydroxy-phenyl)-4-(4-methoxy-phenyl)-1-(3,4,5-trimethoxy-phenyl)-azetidin-2-one* (*22)*** was formed from 3-(3-Benzyloxy-phenyl)-4-(4-methoxy-phenyl)-1-(3,4,5-trimethoxyphenyl)-azetidin-2-one.; lR: NaCl disk u: 1728.60 cm-¹ (C=O, β-lactam); ¹H NMR (400 MHz, CDCl₃) δ3.73 (s, 3H), δ3.79 (s, 3H), δ3.85 (s, 3H), δ4.24 (d, 1 H, J=2.52 Hz), δ4.87 (d, 1H, J=2.48 Hz), δ5.50 (s, 1 H), δ6.60 (s, 2H), δ6.85 - 6.86 (m, 1H). δ6.87 - 6.89 (m, 2H), δ6.95 - 6.97 (m, 2H), δ7.22 - 7.29 (m, 2H), δ7.35 - 7.37 (m, 2H); ¹³C NMR (400 MHz, CDCl₃) δ54.93, δ55.56, δ60.52, δ63.33, δ64.17, δ94.45, δ113.75, δ114.25, δ114.55, δ119.25, δ126.90, δ 128.65, δ129.84, δ133.10, δ135.78, δ153.05, δ155.82, δ159.52, δ165.27; ElMS (HR): C₂₅H₂₆NO₆; mass (+H) 436.1768; calculated mass (+Na) 436.1760; error + 1.8 ppm.

***3-(4-Amino-phenyl)-4-(3-hydroxy-4-methoxy-phenyl)-1-(3,4,5-trimethoxy-phenyl)-azetidin-2-one (23)*** was prepared from {4-[2-(3-Hydroxy-4-methoxy-phenyl)-4-oxo-1-(3,4,5-trimethoxy-phenyl)-azetidin-3-yl]-phenyl}-carbamic acid benzyl ester. It was formed as an orange residue in 39.38% yield; lR: NaCl disk u: 1737.44 cm-¹ (C=O, β-lactam); ¹H NMR (400 MHz, CDCl₃) δ3.75 (s, 6H, -OCH₃), δ3.80 (s, 3H, -OCH₃), δ3.93 (s, 3H, -OCH₃), δ4.18 (d, 1H. J=2.48 Hz, Hₓ), δ4.74 (d, 1H, J=2.48 Hz, Hₓ), δ6.63 (s, 2H, Ar-H), δ6.70 (d, 2H, J=8.56 Hz, Ar-H), δ6.89 (m, 2H, Ar-H), δ6.99 (s, 1H, Ar-H), δ7.11 (d, 2H, J=8 Hz, Ar-H), δ7.29 (s, 1H, - NH₂); ¹³ C NMR (400 MHz, CDCl₃) δ55.56 (-OCH₃), δ55.59 (-OCH₃), δ60.51 (-OCH₃), δ63.85 (Cₓ), δ64.19 (Cₓ), δ94.39 (Ar-C), δ110.54 (Ar-C), δ111.53 (Ar-C), δ115.15 (Ar-C), δ117.33 (Ar-C), δ124.24 (Ar-C), δ128.03 (Ar-C), δ130.32 (Ar-C), δ133.39 (Ar-C), δ145.39 (Ar-C), δ 145.83 (Ar-C), δ146.32 (Ar-C), δ153.02 (Ar-C), δ165.60 (-C=O); ElMS (HR): C₂₅H₂₇N₂O₆; mass (+H) 451.1859; calculated mass (+H) 451.1869; error -2.2 ppm.

***4-(3-Amino-4-methoxy-phenyl)-3-(4-amino-phenyl)-1-(3,4,5-trimethoxy-phenyl)-azetidin-2-one (24)*** was prepared from {4-[2-(4-Methoxy-3-nitro-phenyl)-4-oxo-1-(3,4,5-trimethoxy-phenyl)-azetidin-3-yl]-phenyl}-carbamic acid benzyl ester. It was formed as a yellow powder in 50.09% yield; lR: KBr disk u: 1729.43 cm-¹ (C=O, β-lactam), 3332.00 cm⁻¹ (-NH₂); ¹H NMR (400 MHz, CDCl₃) δ3.73 (s, 6H, -OCH₃), δ3.77 (s, 3H, -OCH₃), δ3.86 (s, 3H, -OCH₃), δ 4.15 (d, 1H, J=2 Hz), δ4.67 (d, 1H. J=2.52 Hz), δ6.63 - 6.76 (m, 8H, Ar-H), δ7.08 (m, 2H, Ar-H), δ7.26 (s, 2H); ¹³C NMR (400 MHz, CDCl₃) δ55.11 (-OCH₃), δ55.58 (-OCH₃), 6 55.69 (-OCH₃), δ60.51, δ64.14, δ94.37 (Ar-C), δ96.98 (Ar-C), δ109.99 (Ar-C), δ111.12 (Ar-C), δ 115.08 (Ar-C), δ115.89 (Ar-C), δ124.39 (Ar-C), δ128.04 (Ar-C), δ129.68 (Ar-C), δ130.21 (ArC), δ133.56 (Ar-C), δ133.83 (Ar-C), δ136.48 (Ar-C), δ152.99 (Ar-C), δ166.11 (-C=O); ElMS (HR): C₂₅H₂₈N₃O₅; mass (+H) 450.2048; calculated mass (+H) 450.2029; error +4_{.}2 ppm

### Synthetic intermediates for compounds 25-28

***2-Methoxy-5-((3,4,5-trimethoxyphenylamino)-methyl]phenol*** A solution of the appropriately substituted aryl aldehyde 3-hydroxy-4-methoxybenzaldehyde (10 mmol, 1.36 g) and the appropriately substituted aryl amine 3,4,5-trimethoxyaniline (10 mmol, 1.37 g) in ethanol (50 ml) was heated to reflux for three hours. The reaction mixture was reduced to 25 ml under vacuum, and the solution transferred to a beaker. The mixture was left to stand and the Schiff base product crystallized out of the solution. The crude product was then re-crystallized from ethanol and filtered to yield the purified product. Yield 84 %, Pale yellow crystals, m.p. 176-178°C. lR vₘₐₓ (KBr) cm-¹: 1602.6 cm-¹ (C=N), 3069.2 cm-¹ (OH). ¹H NMR (400 MHz, DMSO): δ 3.81 (s, 3H, O-CH₃), δ3.84 (d, 9H, J=10.92 Hz, O-CH₃) δ6.57 (s, 2H, Ar-H), δ7.02 (d, 1H, J=8.2 Hz, Ar-H), δ7.31 (q, 1H, J=4.00 Hz, Ar-H), δ7.41 (d, 1H, J=2.04 Hz, Ar-H), δ8.48 (s, 1H. CH=N), δ9.33 (s, 1H, OH). ¹³C NMR (100 MHz, DMSO): δ55.83 (O-CH₃), δ56.09 (O-CH₃), δ 98.50 (Ar-H), δ111.57 (Ar-H), δ113.57 (Ar-H), δ122.26 (Ar-H), δ129.27 (C), δ135.50 (C), δ 146.78 (C), δ147.59 (C), δ150.79 (C), δ153.21 (C), δ159.42 (CH=N). HRMS: Found 318.1345; C,₁₇H₁₉NO₅ requires 318.1341.

***General procedure for protection of of hydroxy group with dimethyl-t-butylchlorosilane*** To a suspension of the appropriate phenol (0.02 mol) and dimethyl-tert butylchlorosilane (0.024 mol) in dry DCM (60 mls) was added 1,8-diazobicyclo[5.4.0] undec-7-ene (DBU) (0.032 mol). The resulting mixture was stirred at room temperature until complete on thin layer chromatography. The solution was then diluted with DCM (80 ml) and washed with water (60 ml), 0.1 M HCI (60 ml) and finally with saturated aqueous NaHC0₃ (60 ml). The organic layer was removed and dried using anhydrous sodium sulphate.

***[3-(tert-Butyidimethylsilanyloxy)-(4-methoxybenzylidene)-(3,4,5-trimethoxy phenyl)-amine** .* Preparation was as above from 2-methoxy-5-[(3,4,5-trimethoxyphenylamino)-methyl]-phenol (0.02 mol, 6.34 g). Yield 91 %, Yellow crystals, m.p. 88-92°C, lR Vₘₐₓ (KBr) cm^{- 1}: 1618.8 cm-¹ (C=N). ¹H NMR (400 MHz, DMSO): δ0.21 (s, 6H, CH₃-Si-CH₃), δ1.04 (s, 9H, Si-C-(CH₃)₃), δ3.74 (s, 3H, O-CH₃), δ3.87 (s, 6H, O-CH₃), δ3.93 (s, 3H, O-CH₃) δ6.58 (s, 2H, Ar-H), δ7.12 (d, 1H, J=8.2 Hz, Ar-H), δ7.48-7.55 (m, 2H, Ar-H), δ8.51 (s, 1H, CH=N). ¹³C NMR (100 MHz, DMSO): δ-5.78 (CH₃-Si-CH₃), δ17.71 (CH₃-C-CH₃), δ24.74 (C-CH₃)₃), δ 55.00 (O-CH₃), δ59.25 (O-CH₃), δ98.04 (Ar-H), δ111.22 (C), δ121.27 (C), δ125.43 (C), δ 129.50 (C), δ135.95 (C), δ 144.68 (C), δ147.73 (C), δ153.32 (C), δ158.27 (CH=N). HRMS: Found 432.2213; C₂₃H₃₃NO₅Si requires 432.2216.

***General preparation of azetidin-2-ones* 25-28** To a suspension of zinc dust (0.9 g, 13.8 mmol) in benzene (20 ml) under nitrogen was added trimethylchlorosilane (0.65 ml, 5 mmol) and the resulting mixture was stirred at room temperature for 15 minutes and then under reflux for a further 2 minutes. The suspension was cooled and the corresponding imine (10 mmol) and ethylbromoacetate (1.33 ml, 12 mmol) were successively added. The reaction mixture was refluxed under nitrogen for 8 hours and then cooled in an ice-water bath. It was then poured over 20 ml of saturated NH₄Cl and 20 ml of 25 % NH₄OH. CH₂Cl₂ (20 ml) is used to extract the organic layer which is further washed with 20 ml 0.1 N HCI and 20 ml of water. The organic layer is separated and dried using anhydrous sodium sulphate. The solvent is evaporated under vacuum and the β-lactam is purified and characterized.

***4-[3-(tert-Butyldimethylsilanyloxy)-4-methoxyphenyl]-1-(3,4,5-trimethoxyphenyl)-azetidin-2-one*** Preparation was as above from ([3-(tert-butyldimethylsilanyloxy)-4-methoxybenzylidene)-(3,4,5-trimethoxyphenyl-amine) (7 mmol, 3.017 g) as described. Yield 21 %, Yellow crystals, m.p. **90-91°C.** lR Vₘₐₓ (KBr) cm⁻¹: 1748.1 **cm⁻¹** (C=O). ¹H NMR (400 MHz, CDCl₃): δ0.06 (d, 6H, J= 8.2 Hz, CH₃-Si-CH₃), δ0.99 (s, 9H, C-(CH₃)₃), δ2.89 (d,d, 1H. J= 2.4 Hz, J=9.6 Hz, H₃), δ3.52 (d,d, 1H, J=6 Hz, 9.2 Hz, H₃), δ3.70 (s, 6H, O-CH₃), δ3.75 (s, 3H, O-CH₃), δ3.79 (s, 3H, O-CH₃), δ4.86, (q, 1H, J=2.72 Hz, H₄), δ6.54 (s, 2H, Ar-H), δ6.81-6.83 (m, 1H, Ar-H), δ6.92-6.95 (m, 1H, Ar-H). ¹³C NMR (100 MHz, CDCl₃): δ-5.13 (CH₃-Si-CH₃), δ 13.65 (CH₃-C-CH₃), δ25.20 (C-(CH₃)₃), δ46.39 C₃, CH₂), δ53.57 (C₄, CH), δ54.91 (O-CH₃), δ 94.04 (Ar-H), δ111.20 (Ar-H), δ115.26 (Ar-H), δ119.05 (Ar-H), δ129.99 (C), δ133.57 (C), δ 143.98 (C), δ150.72 (C), δ152.72 (C), δ164.08 (C=O). HRMS: Found 474.2312; C₂₅H₃₅NO₆Si requires 473.6340.

***4-([3-tert-Butyldimethylsilanyloxy]-4-methoxyphenyl)-3-methyl-1-{3,4,5-trimethoxyphenyl)-azetidin-2-one** .* Preparation was as above from *3-(tert-*butyldimethylsilanyloxy)-(4-methoxyphenyl]-(3,4,5-trimethoxybenzylidene)-amine (10 mmol, 1.8312 g) and (12 mmol, 1.55 ml) of ethyl-2-bromopropionate. Yield 61 %, Brown solid. lR Vₘₐₓ (film) **cm-¹:** 1745.6 **cm-¹** (C=O, β-lactam). ¹H NMR (400 MHz, CDCl₃): δ0.04 (s, 3H, Si-CH₃), δ 0.05 (s, 3H, Si-CH₃), δ0.91 (s, 9H, C-(CH₃)₃), δ1.17 (m, 2H, -CH₃), δ1.26 (m, 1 H, -CH₃), δ 3.12 (d, 0.4H, J=7.5 Hz, H₃), δ3.55-3.58 (m, 0.6H, H₃), δ3.68 (s, 6H, O-CH₃), δ3.73 (s, 3H, δ CH₃), δ3.78 (s, 3H, O-CH₃), δ4.44 (s, 0.4H, H₄), δ5.05 (d, 0.6H, J=5.52 Hz, H₄), δ6.52 (d, 2H, J=3.04 Hz, Ar-H), δ6.54 (s, 1H, Ar-H), δ6.67-6.82 (m, 2H, Ar-H). ¹³C NMR (100 MHz, CDCl₃): δ-5.38- -5.30 (CH₃-Si-CH₃), δ9.05 (-CH₃), δ12.52 (-CH₃), δ17.87 (C-(CH₃)₃) δ28.73 (C-(CH₃)₃), δ48.61 (C₃,CH), δ54.50 (O-CH₃), δ54.87 O-CH₃), δ54.93 (O-CH₃), δ55.36 (C₃,CH), δ 55.39 (O-CH₃), δ 60.32 (C₄,CH), δ62.10 (C₄,CH), δ94.10 (Ar-H), δ94.32 (Ar-H), δ111.48 (Ar-H), δ111.76 (Ar-H), δ110.17 (Ar-H), δ120.08 (Ar-H), δ127.78 (C), δ133.53 (C), δ133.61 (C), δ144.55 (C), δ150.39 (C), δ152.90 (C),δ 167.80 (C=O, C₂), δ167.94 (C=O, C₂).

***4-(3-Hydroxy-4-methoxyphenyl)]-1-(3,4,S-trimethoxyphenyl)-azetidin-2-one* 25.** To a suspension of the appropriately protected phenol above (10 mmol) in THF (50 ml) was added 1.5 equiv of 1 M tetrabutylammonium fluoride. The solution was stirred in an ice bath for 15 minutes to avoid decomposition of the β-lactam ring. The reaction mixture was then diluted with EtOAc (100 ml) and quenched with 10 % HCl (100 ml). The layers are separated and the aqueous layer was extracted with EtOAc (2 x 50 ml). The organic layer was then washed with water (100 ml) and brine (100 ml) and dried with sodium sulphate. Preparation was as above from 4-[3-(tert-butyldimethylsilanyloxy)-4-methoxyphenyl]-1-(3,4,5-trimethoxyphenyl)-azetidin-2-one (0.634 mmol, 0.30 g). Yield 62 %, Yellow gel, lR Vₘₐₓ (film) **cm-¹:** 1746.0 **cm-¹** (C=O), 3404.2 **cm-¹** (OH). ¹H NMR (400 MHz, CDCl₃): δ2.91 (d,d, 1H, J=2.4 Hz, 12.8 Hz, H₃), δ3.48 (d,d, 1 H, J=5.6 Hz, 9.6 Hz, H₃), δ3.70 (s, 6H, O-CH₃), δ3.74 (s, 3H, O-CH₃), δ3.85 (s, 3H, O-CH₃), δ4.86 (q, 1H. J=2.72 Hz, H₄), δ6.54 (s, 2H, Ar-H), δ6.81-6.88 (m, 3H, Ar-H). ¹³C NMR (100 MHz, CDCl₃): δ30.47 (C₃, CH₂), δ46.31 (C₄, CH), δ53.66 (O-CH₃), δ60.44 (O-CH₃), δ 94.01 (Ar-H), δ111.60 (Ar-H), δ117.35 (Ar-H), δ 130.64 (C), δ133.57 (C), δ145.88 (C), δ 146.44 (C), δ152.94 (C), δ164.21 (C=O, C₂). HRMS: Found 360.1454; C₁₉H₂₁NO₆ requires 360.1447.

***4-(4-Methoxyphenyl)-l-(3,4,5-trimethoxyphenyl)-azetidin-2-one* 26**. Preparation was as above from (4-methoxybenzylidene)-3,4,5-trimethoxyphenyl)-amine (5 mmol, 1.5067 g). Yield 43 %, Green crystals, m.p. 70-71°C, lR Vₘₐₓ (film) cm⁻¹: 1747.5 cm⁻¹ (C=O, β-lactam). ¹H NMR (400 MHz, CDCl₃): δ2.85 (d,d, 1H, J= 2.48 Hz, 12.56 Hz, H₃), δ3.48 (d,d, 1H. J=5.52 Hz, J=9.56 Hz, H₃), δ3.65 (s, 6H, O-CH₃), δ3.70 (s, 3H, O-CH₃), δ3.73 (s, 3H, O-CH₃), δ4.88 (d,d, 1H, J= 2.76 Hz, H₄), δ6.53 (s, 2H, Ar-H), δ6.86 (d, 2H, J=8.56 Hz, Ar-H), δ7.26 (d, 2H, J=8.56 Hz, Ar-H). ¹³**C NMR (100 MHz, CDCl₃)**: δ46.36 (C₃, CH₂), δ53.56 (C₄, CH), δ54.78 (O-CH₃), δ 55.23 (O-CH₃), δ55.49 (O-CH₃), δ60.36 (O-CH₃), δ93.92 (Ar-H), δ113.58 (Ar-H), δ126.83 (Ar-H), δ129.48 (C), δ133.62 (C), δ133.68 (C), δ152.94 (C), δ159.29 (C), δ164.14 (C₂, C=O). HRMS: Found 366.1330; C₁₉H₂₁NOₛNa requires 366.1317.

***4-(4-Methoxyphenyl)-3-methyl-1-(3,4,5-trimethoxyphenyl)-azetidin-2-one*** 27. Preparation was as above from (4-methoxybenzylidene)-(3,4,5-trimethoxyphenyl)-amine (5 mmol, 1.5067 g) and (6 mmol, 0.78 ml) of ethyl-2-bromopropionate. Yield 83 %, Dark orange gel, lR Vₘₐₓ (film) cm-¹: 1725.5 cm-¹ (C=O, β-lactam). ¹H NMR (400 MHz, CDCl₃): δ0.75 (d, 2H, J=7.52 Hz, CH₃), δ1.33 (d, 1H, J=7.52 Hz, CH₃), δ3.54 (q, 0.66H, J=6.78 Hz, H₃), δ3.94 (q, 0.34H, J=7.28 Hz, H₃), δ3.58 (d, 6H, J=1 Hz, O-CH₃), δ3.62 (s, 2H, O-CH₃), δ3.64 (s, 4H, O-CH₃), δ4.42 (s, 0.34H, H₄), δ5.01 (d, 0.66H, J=5.6 Hz, H₄), δ6.44 (d, 2H, J=9.04 Hz, Ar-H), δ 6.76 (q, 2H, J=3.67 Hz, Ar-H), δ7.06 (d, 1.33H, J=8.04 Hz, Ar-H), δ7.20 (d, 0.67H, J=8.52 Hz, Ar-H). ¹³C NMR (100 MHz, CDCl₃): δ9.16 (-CH₃), δ12.43 (-CH₃), δ48.65 (C₃,CH), δ55.38 (C₃,CH), δ54.52 (O-CH₃), δ54.58 (O-CH₃), δ54.65 (O-CH₃), δ55.32 (O-CH₃), δ57.76 (C₄,CH), δ60.25 (C₄,CH), δ62.09 (O-CH₃), δ93.98-( Ar-H), δ94.24 (Ar-H), δ113.51 (Ar-H), δ 113.93 (Ar-H), δ126.05 (C), δ126.77 (Ar-H), δ127.67 (Ar-H), δ129.14 (C), δ133.47 (C), δ 152.90 (C), δ158.90 (C), δ167.78 (C=O, C₂), δ167.93 (C=O, C₂). HRMS: Found 380.1473; C₂oH₂₃NO₅Na requires 380.1473.

***4-(3-Hydroxy-4-methoxyphenyl)-3-methyl-1-(3,4,5-trimethoxyphenyl)-azetidin-2-*one 28.** Preparation was as above from 4-([3-*tert*-butyldimethylsilanyloxy)-4-methoxyphenyl)-3-methyl-1-(3,4,5-trimethoxyphenyl)-azetidin-2-one (8 mmol, 3.901 g). Yield 45 %, Brown gel, lR vₘₐₓ (KBr) cm⁻¹: 1724.2 cm⁻¹ (C=O), 3240.2 cm⁻¹ (OH). ¹H NMR (400 MHz, CDCl₃)₁ δ0.91 (d, 2H, J=7.52 Hz, -CH₃), δ1.44 (d, 1H, J=7 Hz, -CH₃), δ3.09-3.14 (d,q, 0.33H, J=2.24 Hz, 5.02 Hz, H₃), δ3.59-3.67 (d,q, 0.67H, J=7.52 Hz, 6.02 Hz, H₃), δ3.72 (d, 6H, J=2.52 Hz, O-CH₃), δ 3.76 (s, 1H, O-CH₃), δ3.78 (s, 2H, O-CH₃), δ3.89 (s, 3H, O-CH₃), δ4.44 (d, 0.33H, J=2 Hz, H₄), δ5.07 (d, 0.67H, J=5.6 Hz, H₄), δ5.81 (bs, 1H. OH), δ6.55 (s, 0.67H, Ar-H), δ6.57 (s, 1.33H, Ar-H), δ6.72 (q, 1H, J=2.02 Hz, 6.52 Hz, Ar-H), δ6.82 (t, 1H, J=2.5 Hz, Ar-H, δ6.85 (q, 1H, J=2.33 Hz, Ar-H). ¹³C NMR (100 MHz, CDCl₃): δ9.15 (-CH₃), δ12.62 (-CH₃), δ45.51 (C₃, CH), δ 54.61 (C₃, CH), δ 55.47 (O-CH₃), δ 55.56 (O-CH₃), δ 56.62 (O-CH₃), δ 57.90 (C₄, CH), δ 62.31 (C₄, CH), δ 94.14 (Ar-H), δ 94.37 (Ar-H), δ 110.17 (Ar-H), δ 110.49 (Ar-H), δ 112.71 (Ar-H), δ 118.24 (Ar-H), δ 127.36 (C), δ 130.48 (C), δ 133.64 (C), δ 145.33 (C), δ 146.29 (C), δ 152.98 (C), δ 167.94 (C=O, C₂), δ 168.10 (C=O, C₂). HRMS: Found 396.1419; C₂₀H₂₃NO₆ requires 396.1423.

Table 2 illustrates the results of the NCl60 cell line screen results for compounds 4, 8 and 12 given in Table 1 above.

**Table 2: NCl60 cell line screen results for compounds 4, 8 and 12.**

| **Panel** | **Cell line** | **Gl₅₀ (µM) 8** | **Gl₅₀ (µM) 12** | **Gl₅₀(µM) 4** |
|---|---|---|---|---|
| Leukemia | CCRF-CEM | 0.0372 | <0.010 | <0.010 |
| | HL-60(TB) | 0.0162 | <0.010 | <0.010 |
| | K-562 | 0.0291 | nd | <0.010 |
| | MOLT-4 | 0.0316 | nd | nd |
| | RPMI-8226 | 0.0293 | <0.010 | <0.010 |
| NSCLC | A549/ATCC | 0.238 | 0.023 | <0.010 |
| | EKVX | 0.352 | 0.034 | <0.010 |
| | HOP-62 | 0.605 | 0.070 | nd |
| | HOP-92 | 22.700 | 0.051 | 5.15 |
| | NCl-H226 | 0.263 | <0.010 | <0.010 |
| | NCl-H23 | 0.427 | 0.036 | <0.010 |
| | NCI-H332M | 0.298 | 0.027 | <0.010 |
| | NCl-H460 | 0.198 | 0.030 | <0.010 |
| | NCl-H552 | 0.0966 | <0.010 | 5.15 |
| Colon | COLO 205 | 0.166 | 0.015 | 0.019 |
| | HCT-2998 | 0.349 | 0.030 | 0.035 |
| | HCT-116 | 0.0398 | <0.010 | <0.010 |
| | HCT-15 | 0.170 | 0.026 | <0.010 |
| | KM12 | 0.0427 | <0.010 | <0.010 |
| | SW-620 | 0.0487 | 0.010 | <0.010 |
| CNS | SF-268 | 0.354 | 0.028 | <0.010 |
| | SF-295 | 0.035 | <0.010 | <0.010 |
| | SF-539 | 0.033 | <0.010 | <0.010 |
| | SNB-19 | 0.280 | 0.043 | 0.025 |
| | SNB-75 | nd*^{a}* | nd | 0.047 |
| | U251 | 0.095 | 0.037 | <0.010 |
| Melanoma | LOX lMVl | 0.0676 | <0.010 | <0.010 |
| | MALME-3M | > 100 | nd | nd |
| | M14 | 0.132 | <0.010 | <0.010 |
| | SK-MEL-2 | > 100 | 7.79 | 0.014 |
| | SK-MEL-28 | 0.405 | 0.010 | <0.010 |
| | SK-MEL-5 | 0.124 | 0.014 | <0.010 |
| | UACC-257 | 27.9 | 67.8 | 22.4 |
| | UACC-62 | 0.0405 | <0.010 | <0.010 |
| Ovarian cancer | lGROV1 | 0.285 | 0.020 | <0.010 |
| | OVCAR-4 | 0.0825 | 0.013 | 0.018 |
| | OVCAR-5 | 0.668 | 0.051 | 0.038 |
| | OVCAR-8 | 0.125 | <0.010 | 0.010 |
| | SK-OV-3 | 0.412 | 0.038 | 0.037 |
| Renal cancer | 786-0 | 0.432 | 0.031 | 0.034 |
| | A498 | 0.0375 | <0.010 | <0.010 |
| | ACHN | 0.138 | <0.010 | <0.010 |
| | CAKl-1 | 0.0848 | 0.016 | <0.010 |
| | RXF 393 | 0.0365 | <0.010 | <0.010 |
| | SN12C | 0.312 | <0.010 | <0.010 |
| | TK-10 | 6.78 | <0.010 | <0.010 |
| | UO-31 | 0.396 | 0.038 | 0.032 |
| Prostate cancer | PC-3 | nd*^{a}* | <0.010 | <0.010 |
| | DU-145 | 0.0569 | <0.010 | <0.010 |
| Breast cancer | MCF-7 | 0.0351 | <0.010 | <0.010 |
| | NCl/ADR-RES | 0.0425 | <0.010 | <0.010 |
| | MDA-MB-231/ATCC | 0.200 | 0.010 | <0.010 |
| | MDA-MB-435 | 0.0239 | <0.010 | <0.010 |
| | BT-549 | 0.117 | <0.010 | <0.010 |
| | T-47D | > 100 | >1000 | 31.2 |
| | MDA-MB-468 | 0.0684 | <0.010 | <0.010 |

| | | | | |
|---|---|---|---|---|
| a. nd = not done | | | | |

As can be seen from Table 3 compounds of the present invention also potently inhibit the growth of human breast carcinoma MCF-7 cells, human chronic myeloid leukaemia K562 cells and human promyelocytic leukaemia HL-60 cells in an MTT cell viability assay with lC50 values in the nanomolar or subnanomolar range.

**Table 3: Effect of CA-4 and novel CA's on cell viability of human MCF-7 breast cancer cells, human promyelocytic HL-60 leukemia and human K562 chronic myeloid leukaemia cells**

| **Compound** | **Inhibition of cell viability (IC50 value, nM)** | | |
|---|---|---|---|
| | **MCF-7** | **HL-60** | **K562** |
| **CA-4 (Control)** | 3.1 | 4.1 | 3.8 |
| **2** | 1.4 | 17.1 | 26.6 |
| **14** | 0.8 | 0.4 | 0.9 |

Compounds **2** and **14** are more potent than CA-4 at inhibiting human MCF-7 cell growth and **14** is 10-fold more potent than CA-4 at inhibiting HL-60 promyelocytic leukaemia cell growth. Independent verification of the antiproliferative results for a small number of compounds was obtained by evaluation by NCl (NlH) screening programme for anticancer activity in a 60 cell line screen. Comprehensive data for selected compounds 4, 8 and 12 in 60 cell lines showed lC₅₀ values <10nm in 32 of the 60 cell lines (for some of the compounds) examined in NCl programme.

In addition, *in vitro* stability studies of the lead compounds in plasma (carried out in our laboratory) have indicated a half-life greater than 24 hours. For example, the half life for compound 1 in human plasma is 35 hours. Furthermore, preliminary results demonstrate that the compounds of the present invention elicit minimal toxicity in primary normal mammary epithelial cells (see Table 4). As can be seen from Table 4, both CA-4 and compounds 2 and 14 elicit minimal effects on normal mammary epithelial cells. IC50 values for inhibition of cell viability could not be obtained as even the highest concentration of drug tested (10µM) only reduced cell viability to a maximum of 75% of the control. The lC50 values of compounds 2 and 14 for inhibiting MCF-7 human breast carcinoma cells range from 0.8-3.1 nM (see Table 3). The maximum toxicity elicited by these drugs on normal cells at a concentration of 10 nM was only 8% (see Table 4).

**Table 4: Effects on normal mouse mammary epithelial cells**

| | **Percent viability of normal mammary epithelial cells compared to control following 72hr treatment with indicated drugs** | | | | |
|---|---|---|---|---|---|
| Drug Concentration | 1 nM | 10 nM | 100 nM | 1 µM | 10 µM |
| CA-4 | 98 | 92 | 74.8 | 74.7 | 75.0 |

| (Control) | | | | | |
|---|---|---|---|---|---|
| 2 | 93.1 | 92.3 | 74.4 | 73.0 | 73.8 |
| 14 | 93.8 | 90 | 73.2 | 75.2 | 74.8 |

### Biological Testing

### Antiproliferation studies.

All assays were performed in triplicate for the determination of mean values reported. Compounds were assayed as the free bases isolated from reaction. The human breast tumour cell line MCF-7 was cultured in Eagles minimum essential medium in a 95%O₂/5% CO₂ atmosphere with 10% fetal bovine serum, 2 mM L-glutamine and 100 µg/mL penicillin/streptomycin. The medium was supplemented with 1% non-essential amino acids. MDA-MB-231 cells were maintained in Dulbecco's Modified Eagle's medium (DMEM), supplemented with 10% (v/v) Fetal bovine serum, 2mM L-glutamine and 100 µg/mL penicillin/streptomycin (complete medium). Cells were trypsinised and seeded at a density of 2.5 x 10⁴ cells/ml in a 96-well plate and incubated at 37°C, 95%O₂/5% CO₂ atmosphere for 24 h. After this time they were treated with 2 µL volumes of test compound which had been pre-prepared as stock solutions in ethanol to furnish the concentration range of study, 1 nM-100 µM, and re-incubated for a further 72 h. Control wells contained the equivalent volume of the vehicle ethanol (1% v/v). The culture medium was then removed and the cells washed with 100 µL phosphate buffered saline (PBS) and 50 µL MTT added, to reach a final concentration of 1 mg/mL MTT added. Cells were incubated for 2 h in darkness at 37°C. At this point solubilization was begun through the addition of 200 µL DMSO and the cells maintained at room temperature in darkness for 20 min to ensure thorough colour diffusion before reading the absorbance. The absorbance value of control cells (no added compound) was set to 100 % cell viability and from this graphs of absorbance versus cell density per well were prepared to assess cell viability and from these, graphs of percentage cell viability versus concentration of subject compound added were drawn.

### Cell cycle analysis:

*Flow cytometry.* The MDA-MB-231 cells were seeded at a density of 18 x 10⁴ cells/mL in 5 mL of medium (900,000 cells per flask). After 24 hours the control was treated with 50 µL of ethanol (1 % v/v) and selected compound dosed at range 10 nM -10 µM (final concentration, 1 % v/v). They were incubated for 72 hours. Following incubation, the cells were removed from the bottom of the flask by scraping and the medium placed in a 20 mL sterilin. They were centrifuged for 10 minutes at 600xg. The supernatant was decanted and the pellet resuspended in 1 mL of ice-cold PBS; cells were again centrifuged for 10 minutes at 600xg. The supernatant was decanted and the pellet resuspended in 200 µL of ice-cold phosphate buffer saline (PBS). Subsequently ice-cold 70 % ethanol (2 mL) was slowly added to the tube as it was gently vortexed. The cells were kept at -20°C for at least one hour. After the fixation 5 µL of FBS was added to the samples. The cells were harvested by centrifugation at 600xg for 10 mins. The ethanol was carefully removed and the pellet resuspended in 400 µL of PBS and transferred to FACS microtubes. A 25 µL aliquot of RNase A (1 mg/mL) and 75 µL of propidium iodide (PI) 1 mg/mL, a DNA binding fluorescent dye, was added to each tube. The samples were wrapped in aluminium foil and incubated for a minimum of 30 min at 37°C. The samples were read at 488 nM using FACscalibur flow cytometer from Becton Dickinson. The FACS data for 10,000 cells was analysed using the Macintosh-based application Cellquest and the data was stored as frequency histograms.

### Evaluation of G₂/M arrest in MDA-MB-231 cells exposed to compound 18d

Compound 25 showed antiproliferative effects at low nanomolar concentrations (17 nM, MCF-7 and 54 nM, MDA-MB-231) therefore flow cytometric analysis was performed as a means of statistically quantifying the extent of G₂/M arrest and sub-G₁ arrest induced by compound 25 in MDA-MB-231 cells. The fluorescent dye, Pl interchelates with the DNA and hence, the amount of fluorescence measured per cell is proportional to the DNA content. MDA-MB-231 cells were treated with vehicle (1 % (v/v) ethanol) or 10 nM, 100 nM, 1 µM and 10 µM (final concentrations) of 25. Cells were harvested after 24, 48 or 72 hours and analysed for DNA content by flow cytometry.

Tables 7, 8 and 9 show the percentage of cells in each phase of the cell cycle over the three different time scales.

**Table 7: % MDA-MB-231 cells in each cell cycle phase after exposure to compound 25 for 24 hours. Values represent the mean ± standard deviation for three experiments.**

| **Concentration** | **Sub-G₁ (%)** | **G₁ (%)** | **S (%)** | **G₂/M (%)** | **M5(%)** |
|---|---|---|---|---|---|
| Control | 5.71 ± 3.47 | 51.20 ± 2.37 | 10.46 ± 4.19 | 20.18 ± 3.18 | 4.53 ± 1.66 |
| 10 nM | 3.72 ± 1.97 | 48.33 ± 4.18 | 12.55 ± 2.54 | 25.61 ± 0.98 | 2.84 ± 0.59 |
| 100 nM | 4.88 ± 0.85 | 18.95 ± 6.60 | 6.05 ± 0.38 | 59.43 ± 3.13 | 4.87 ± 4.39 |
| 1 µM | 7.08 ± 4.20 | 19.22 ± 6.19 | 6.01 ± 2.90 | **63.16 ± 4.86** | 4.31 ± 3.17 |
| 10 µM | 3.18 ± 0.25 | 29.30 ± 5.95 | 3.72 ± 0.71 | 55.36 ± 1.49 | 3.85 ± 3.83 |

**Table 8: % MDA-MB-231 cells in each cell cycle phase after exposure to compound 25 for 48 hours. Values represent the mean ± standard deviation for three experiments.**

| Concentration | Sub-G₁ (%) | G₁ (%) | S (%) | G₂/M (%) | M5(%) |
|---|---|---|---|---|---|
| Control | 9.80 ± 1.55 | 50.34 ± 11.09 | 10.50 ± 0.77 | 18.81 ± 1.04 | 5.62 ± 3.06 |
| 10 nM | 11.57 ± 3.06 | 45.25 ± 7.37 | 13.36 ± 2.58 | 23.71 ± 7.81 | 6.10 ± 0.62 |
| 100 nM | 37.50 ± 0.71 | 10.76 ± 0.34 | 8.90 ± 0.85 | 36.42 ± 3.42 | 4.61 ± 0.86 |
| 10 µM | 51.27 ± 6.31 | 14.44 ± 2.28 | 4.47 ± 0.06 | 27.25 ± 4.45 | 2.28 ± 0.06 |

**Table 9: % MDA-MB-231 cells in each cell cycle phase after exposure to compound 25 for 72 hours. Values represent the mean ± standard deviation for three experiments.**

| Concentration | Sub-G₁ (%) | G₁ (%) | S (%) | G₂/M (%) | M5 (%) |
|---|---|---|---|---|---|
| Control | 12.92 ± 2.61 | 44.13 ± 2.31 | 9.10 ± 2.75 | 21.51 ± 4.86 | 5.48 ± 3.26 |
| 10 nM | 15.25 ± 2.03 | 48.24 ± 2.79 | 9.08 ± 2.32 | 17.68 ± 4.58 | 3.55 ± 1.57 |
| 100 nM | 63.10 ± 20.06 | 13.90 ± 3.62 | 5.34 ± 4.32 | 12.62 ± 9.22 | 2.99 ± 1.89 |
| 1 µM | 50.53 ± 14.78 | 14.29 ± 2.20 | 7.76 ± 0.58 | 21.24 ± 12.05 | 5.97 ± 4.71 |
| 10 µM | 46.49 ± 17.49 | 17.14 ± 3.31 | 9.66 ± 1.84 | 21.81 ± 12.40 | 3.38 ± 2.84 |

The results obtained for compound 25 show a large build up of cells in the G₂/M phase (bolded text) at concentrations 100 nM, 1 µM and 10 µM after 24 hours (Table 7) of exposure. This increase is accompanied by a corresponding reduction in the G₁ phase. At a concentration of 10 nM, there appears to be little effect on the cells giving results similar to the vehicle value. After 48 hours (Table 8) an increase in G₂/M arrest (bolded text) is again seen at 100 nM and above but not to the same degree as seen after 24 hours. This is due to the parallel increase in sub-G₁ phase (bolded text) indicating induction of apoptosis. After 72 hours (Table 9), a decrease in G₂/M and in G₁ phase is accompanied by a large increase in sub-G₁ phase (bold text). These results indicate that this compound's mechanism of action may indeed be by targeting the microtubules. The ability of these β-lactam compounds to bind to tubulin was then investigated.

### Tubulin polymerization assay:

The effect of compounds on the assembly of purified bovine brain tubulin was determined spectrophotometrically by monitoring the change in turbidity. This assay used a 96-well plate format with 300 µg of > 99 % purified bovine brain tubulin in each well. Lyophilised tubulin (1 mg, Cytoskeleton, Denver, CO) was resuspended on ice in 300 µl in ice-cold G-PEM buffer (80 mM PIPES pH 6.9, 0.5 mM MgCl₂, 1 mM EGTA, 1 mM Guanidine Triphosphate (GTP), 10.2 % (v/v glycerol)) and was left on ice for 1 minute to allow for complete resuspension. 10 µl of 10X strength of each compound tested was pipetted into a half area 96-well plate prewarmed to 37°C. A 100 µl volume of tubulin was then pipetted into the prewarmed plate. Samples were mixed well and tubulin assembly was monitored at an A340 nm at 30 second intervals for 60 minutes at 37°C in a Spectromax 340PC spectrophotometer (Molecular Devices).

The effects of combretastatin A-4 and compound 25 on the assembly of purified bovine tubulin were evaluated. Compound 25 demonstrated potent antiproliferative effects (low nanomolar) *in vitro.* The ability of combretastain A-4 to effectively inhibit the assembly of tubulin was assessed as a control. As anticipated the active combretastatin A-4 analogue (25) inhibited the polymerisation of tubulin (Table 10). In more detail, the active combretastatin analogues [10 µM] reduced the Vmax value for the rate of tubulin polymerisation from 6 to 10-fold. This value is comparable if not superior to the rate of inhibition of tubulin assembly (6-fold) observed with combretastatin A-4. These results suggest that the molecular target of the active ß-lactam combretastatin A-4 analogues may be tubulin.

**Table 10: X-fold inhibition of tubulin polymerisation of β-lactam combretastastin analogues**

| Structure | Compound | X-fold inhibition of tubulin polymerisation (Vmax ± SEM) |
|---|---|---|
| Control | Ethanol [1% v/v] | 1 |
| 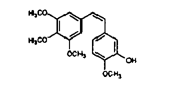 | CA-4 - 10 µM | 6.0 ± 1.4 |
| 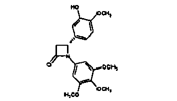 | 25 - 1 µM 25 - 10 µM | 1.1 ± 0.2 10.2 ± 2.3 |

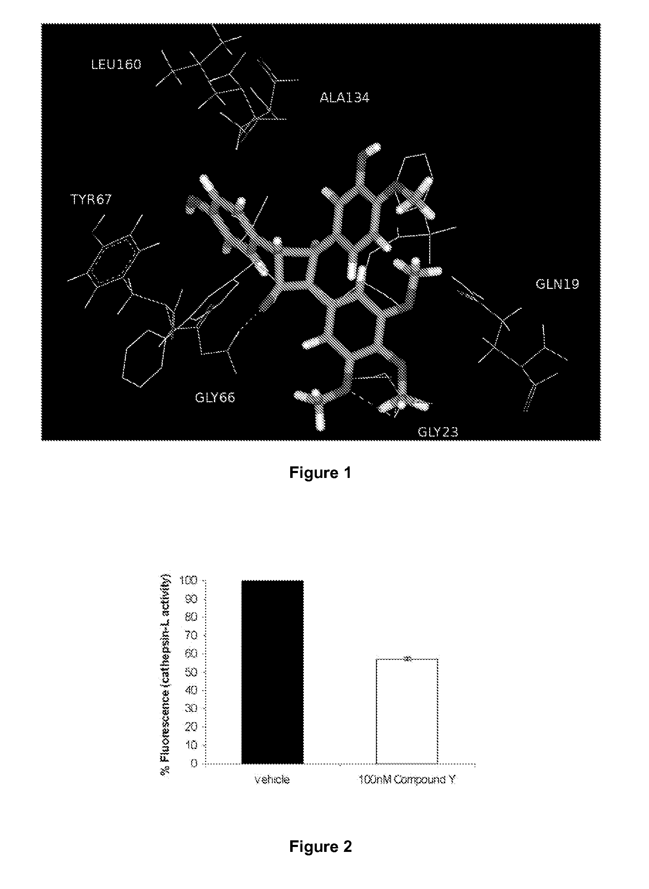

Many cathepsins such as B, K, L and S have been shown to be overexpressed in many tumour types and play a role in cancer metastasis through degradation of the basement membrane and extracellular matrix surrounding the tumour. In addition, inhibition of cathepsin function has been shown to impair tumour development. Consequently cathepsins are important targets for the development of inhibitors as therapeutic agents. We have preliminary evidence that the compounds of the present invention, due to their β-lactam pharmacophore, inhibit tumour cathepsin activity (see Figure 1), which potentially would help to limit tumour metastases. The dual targeting of both tubulin and cathepsins in tumour cells by these novel CA analogues should greatly enhance the overall anti-cancer efficacy of these compounds.

*In silico* molecular modelling examined the docking of the β-lactam compounds in the active site of the cysteine protease cathepsin K. Inspection of the active site of cathepsin K from X-Ray structure predicts good cathepsin K inhibitor activity for the β-lactam compounds of the present application.

Figure 1 shows the docking of compound 14 in the active site of cathepsin K. Interactions of the β-lactam with key active site residues Cys25, Gly66, Tyr67, Leu160, Gln19 and Ser 24 are present, (the hydrogen bonding interactions are shown as broken lines). The interaction of the β-lactam compound 14 at active site residues correlates well with interaction of known cathepsin inhibitors. The coordinates for the complex of cathepsin K are deposited in the Brookhaven Protein Data Bank, accession number 1 BGO and were accessed for the modelling study.

Cathepsin L activity was measured using the flurogenic cathepsin-L activity kit from calbiochem which uses purified human liver cathepsin-L as the source of the cathepsin. As can be seen from Figure 2 compound 14 inhibited cathepsin-L in the nanomolar range (indicated as compound Y in Figure 2). Values represent the mean +/- range of two separate experiments each carried out in duplicate.

### Multi-Drug Resistance

The anti-proliferative effects of CA-4 and compound 4 were evaluated in multi-drug resistant cells. Three drug resistant cell lines and respective parental cell lines were assessed. Specifically, we exposed HL-60-parental, HL-60-MDR (overexpress p-glycoprotein), HL-60-BCRP (breast cancer resistant protein), A2780-parental, A2780-ADR (overexpress p-glycoprotein) to CA-4, compound 4 and selected drugs required to confirm drug resistance. Western blot analysis confirmed the overexpression of p-glycoprotein and BCRP in respective cell lines. P-glycoprotein and BCRP are drug efflux transporters of the ATP binding cassette (ABC) family of proteins. Levels of p-glycoprotein have been shown to correlate with paclitaxel resistance *in vitro.* As shown in Table 11, the calculated resistant factors (RF) demonstrate that neither CA-4 nor compound 4 display cross-resistance with other microtubule-targeting agents; paclitaxel and vincristine, or adriamycin. Taken together, these results suggest that unlike paclitaxel and vincristine, CA-4 and compound 4 are poor substrates for the p-glycoprotein.

Cells were exposed to multiple concentrations of the indicated compound for 72 h. Cell viability was assessed using the Alamar blue assay and respective lC50 values were calculated. The resistance factor (RF) was calculated by dividing the lC50 of the resistant cell line/lC50 of the parental cell line.

K562 and HL-60 cells were originally obtained from the European Collection of Cell Cultures (Salisbury, UK). HL-60-BCRP and HL-60-MDR cells were generously provided by The Hungarian Academy of Sciences, Budapest, Hungary. A2780-parental and A2780-ADR resistant cells were provided by the Beatson Institute of Cancer Research, Glasgow. The K562 cells were derived from a patient in the blast crisis stage of CML. HL-60 cells were derived from a patient with acute myeloid leukaemia. Peripheral blood mononuclear cells were isolated from herparinised peripheral blood of CML patients by Lymphoprep™ (Axis-Shield, Norway) density gradient centrifugation. All cells were cultured in RPMl-1640 Glutamax medium supplemented with 10% FCS media, 100 units/ml penicillin and 100 µg/ml streptomycin. Cells were maintained at 37°C in 5% CO₂ in a humidified incubator. Cell culture materials were supplied from Gibco, Invitrogen Corp (Grand Island, NY, USA).

Alamar Blue cell viability assay: The cytotoxic effects of combretastatin-A4 (CA-4) and selected ß-lactam analogues on leukaemia cells were determined using the Alamar Blue assay (Invitrogen Corp). The reduction of Alamar Blue is proportional to the number of viable cells. Cells (200 µl) were plated in triplicate in a 96-well plates (K562, 100,000/ml; HL-60, 300,000/ml; A-2780 50,000/ml). A-2780 cells were plated 24 h prior to treatment. Suspension cells were plated in the log phase of growth and treated immediately. The cells were then treated with either medium alone, vehicle (1% ethanol v/v or 0.1 % DMSO v/v) or with a range of concentrations of drug [0.001-10 µM]. After 72 h, Alamar Blue was added to each well (10% of final volume) and fluorescence was read using a 96-well fluorometer with excitation at 530 nm and emission of 590 nm. The blank solution consisted of medium and Alamar Blue and the blank solution was used to calibrate the spectrophotometer to zero absorbance. The relative cell viability (%) related to control wells and was calculated by [A]ₜₑₛₜ/ [A]_{control} x 100 where [A]ₜₑₛₜ is the absorbance of the drug treated cells and [A]_{control} is the absorbance of the vehicle control treated cells. Dose response curves were plotted and lC50 values (concentration of drug resulting in 50% reduction in cell survival) were obtained using the commercial software package Prism (GraphPad Software, Inc., La Jolla, USA). Experiments were performed in triplicate on at least three separate occasions.

**Table 11**

| **Cell line** | **compound** | **IC50 [nM]** | **RF** |
|---|---|---|---|
| | | | |
| HL-60 parental | CA-4 | 4.5 | |
| | Compound 4 | 17.7 | |
| | Paclitaxel | 4.8 | |
| | Vincristine | 2.2 | |
| | | | |
| HL-60-MDR(PGP) | CA-4 | 3.3 | 0.73 |
| | Compound 4 | 18.2 | 1.03 |
| | Paclitaxel | > 10,000 | >2,083 |
| | vincristine | 610 | 277.3 |
| | | | |
| HL-60-BCRP | CA-4 | 3.5 | 0.8 |
| | Compound 4 | 25.3 | 1.4 |
| | | | |
| A2780-parental | CA-4 | 7.5 | |
| | Compound 4 | 23.3 | |
| | Paclitaxel | 4.2 | |
| | Vincristine | 3.1 | |
| | Adriamycin | 42.8 | |
| | | | |
| A2780-ADR (PGP) | CA-4 | 6.8 | 0.9 |
| | Compound 4 | 10.4 | 0.5 |
| | Paclitaxel | > 10,000 | >2,381 |
| | Vincristine | 1,600 | 516 |
| | Adriamycin | 5,100 | 119.2 |

*CA-4 and Compound 4 induce apoptosis in ex vivo imatinib mesylate naive and resistant BCR-ABL-positive chronic myelgenous leukaemia (CML) cells.*

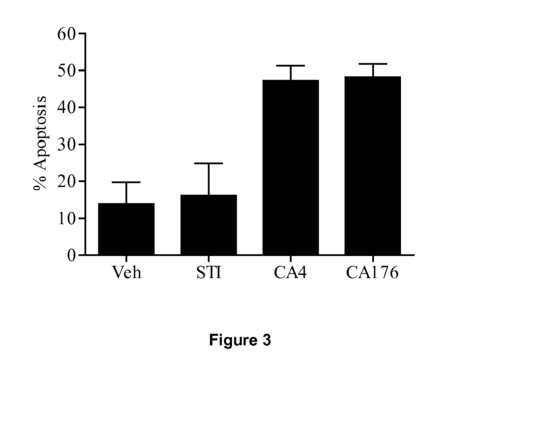

This study is the first pre-clinical evaluation of combretastatins in the treatment of chemotherapy naive and patients with acquired imatinib mesylate resistance (refractory or relapsed disease despite adequate dose and duration). Imatinib mesylate is the first line treatment in BCR-ABL-positive CML chemotherapy. BCR-ABL, a constitutively active protein tyrosine kinase is a product of the Philadelphia chromosome (Ph) translocation t(9;22) and plays a central role in the pathogenesis of CML. The apoptotic potency of CA-4 and compound 4 (indicated as CA176 in Figure 3) with imatinib mesylate in primary CML cells was compared. As shown in Figure 3, both CA-4 and compound 4 induced apoptosis in chemotherapy naïve (4 patient samples) and imatinib mesylate resistant (2 patient samples) primary CML cells. All cells were treated with a clinically achievable concentration of imatinib mesylate (indicated as STl in Figure 3) [250 nM]. For comparison, both CA-4 and compound 4 were used at the same concentration. Interestingly, CA-4 and compound 4 were more effective inducers of apoptosis than imatinib mesylate in the patient samples tested. The percentage apoptosis was determined by flow cytometric analysis of annexin-V stained cells.

### Flow cytometric cell cycle analysis

The flow cytometric evaluation of cellular DNA content was performed as follows. Briefly, after treatment cells were fixed in 70% ethanol, treated with RNase A, and stained with propidium iodide (Pl). The Pl fluorescence was measured on a linear scale using a FACSCalibur flow cytometer (Becton Dickinson, San Jose, CA). The amount of Pl fluorescence is directly proportional to the amount of DNA present in each cell. Data collection was gated to exclude cell debris and cell aggregates. At least 10,000 cells were analysed per sample. All data were recorded and analysed using the CellQuest software (Becton Dickinson).

### Annexin V staining

The percentage of apoptosis in *ex-vivo* CML cells was determined by annexin V staining. PBMCs (1x10⁶) were treated with vehicle or 250 nM of CA-4, CA-176 or imatinib myesylate for 72 h. Cells were collected by centrifugation at 400 x g for 5 min and resuspended in anti-CD-45 diluted 1:50 in RPMl medium. Following a 10 min incubation in the dark at room temperature, cells were centrifuged and washed in Annexin binding buffer (Biosource, Nivelles, Belgium). Cells were again centrifuged and resuspended in Annexin-V-FITC (IQ products, Netherlands) diluted in Annexin binding buffer (1:50). Samples were next incubated in the dark on ice for 15 min. Annexin binding buffer (1ml) was added to each sample. Samples were collected by centrifugation and resuspended in 0.5 ml of Annexin binding buffer. Cells were read immediately by flow cytometry and analysed by Cellquest software. CML cells were selected and gated based on their low to medium side scatter and low CD45 expression.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination.

## Claims

1. A compound of the general formula: 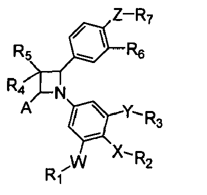 a tautomer thereof, a pharmaceutically acceptable salt thereof, or a hydrate thereof,
wherein W, X, Y and Z may be the same or different and may be selected from the group consisting of CH₂, O, S and NH;
R₁ to R₃ and R₇ may be the same or different and may be C₁-C₅ alkyl;
R₆ may be selected from the group consisting of hydrogen, amino, hydroxy, thiol, cyano, halogen, nitro, OC(O)R₈, SC(O)R₈, NC(O)R₈, OP(O)(OR₉)(ORio) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from H, =O, =S;
R₄ may be selected from the group consisting of hydrogen, hydroxy, amino, and halogen; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy;
such that when R₅ is hydrogen R₄ is not hydroxy.

2. A compound according to Claim 1 of the general formula: 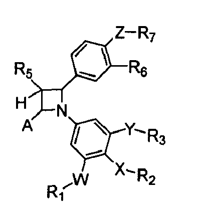 a tautomer thereof, a pharmaceutically acceptable salt thereof, or a hydrate thereof,
wherein W, X, Y and Z are the same or different and are selected from the group consisting of O, S and NH;
R₁ to R₃ and R₇ are the same or different and are C₁-C₅ alkyl;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂ₒ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from =O and =S; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

3. A compound according to Claim 1 or 2 wherein W, X, Y and Z are O and R₁ to R₃ and R₇ are Me.

4. A compound according to Claim 1 or 2 of the general formula: 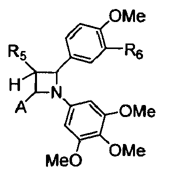 a tautomer thereof, a pharmaceutically acceptable salt thereof, or a hydrate thereof,
wherein A may be selected from =O and =S;
R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H₁ C₁-C₂₀ aliphatic, C₃-C₂₀ cycloaliphatic, C₅-C₂₀ aryl, C₃-C₂₀ heteroaryl, metal cations, and polyatomic cations; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

5. A substantially enantiopure molecule of the of the general formula: 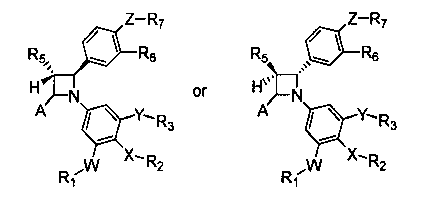 a tautomer thereof, a pharmaceutically acceptable salt thereof, or a hydrate thereof,
wherein W, X, Y and Z may be the same or different and may be selected from the group consisting of CH₂, O, S and NH;
R₁ to R₃ and R₇ may be the same or different and may be C₁-C₅ alkyl; R₆ may be selected from the group consisting of hydrogen, amino, hydroxy, thiol, cyano, halogen, nitro, OC(O)R₈, SC(O)R₈, NC(O)R₈, OP(O)(OR₉)(OR₁₀) and combinations thereof, wherein
R₈ may be selected from the group consisting C₁-C₂₀ aliphatic, C₃-C₂₀ cycloaliphatic, C₅-C₂₀ aryl, C₃-C₂₀ heteroaryl and combinations thereof;
R₉ and R₁₀ may be the same or different and may be selected from the group consisting of H, C₁-C₂₀ aliphatic, C₃-C₂₀ cycloaliphatic, C₅-C₂₀ aryl, C₃-C₂₀ heteroaryl, metal cations, and polyatomic cations;
A may be selected from =O and =S; and
R₅ may be selected from the group consisting of hydrogen, C₅-C₂₀ aryl, C₃-C₂₀ heteroaryl, C₁-C₂₀ aliphatic optionally having at least one C-C unsaturated bond in the chain, C₃-C₂₀ cycloaliphatic optionally having at least one C-C unsaturated bond in the ring, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, halogen and combinations thereof, optionally substituted one or more times with at least one of hydroxy, amino, halogen, cyano, C₁-C₅ alkoxy, and C₁-C₅ thioalkoxy.

6. A compound according to any preceding Claim wherein R₅ is selected from the group consisting of C₂-C₂₀ aliphatic having at least one C-C unsaturated bond in the chain, C₃-C₂₀ cycloaliphatic having at least one C-C unsaturated bond in the ring, C₅-C₂₀ aryl, C₃-C₂₀ heteroaryl, C₅-C₂₀ aryloxy, C₃-C₂₀ heteroaryloxy, C₂-C₅ alkoxy having C-C unsaturated bonds in the alkyl chain, C₂-C₅ thioalkoxy having C-C unsaturated bonds in the alkyl chain, and combinations thereof, optionally substituted one or more times with at least one of hydroxy, amino, halogen, cyano, C₁-C₅ alkoxy, and C₁-C₅ thioalkoxy.

7. A compound according to any preceding Claim wherein R₆ is selected from the group consisting of hydrogen, amino, and hydroxy.

8. A pharmaceutical composition comprising a compound according to any preceding claim, a tautomer thereof, a pharmaceutically acceptable salt thereof, or a hydrate thereof together with a pharmaceutical acceptable carrier or excipient.

9. A compound according to any preceding Claim, a tautomer thereof, a pharmaceutically acceptable salt thereof, or a hydrate thereof for use in the treatment of a disorder that involves vascular proliferation.

10. A compound according to Claim 9 wherein the disorder is a cancer.

11. A compound according to Claim 10 wherein the cancer is a metastatic cancer.

12. A compound according to Claim 10 wherein the cancer is selected from the group consisting of leukaemia, lymphoma, non-small cell lung cancer, colon, central nervous system, melanoma, ovarian, renal, prostate, pancreatic, liver, bone, cervical and breast cancer.

13. A compound according to any one of Claims 1 to 7, a tautomer thereof, a pharmaceutically acceptable salt thereof, or a hydrate thereof for the inhibition of tubulin formation.

14. A compound according to any one of Claims 1 to 7, a tautomer thereof, a pharmaceutically acceptable salt thereof, or a hydrate thereof for use in the treatment of a disorder associated with cathepsin protease activity.

15. A compound according to Claim 14 wherein the cathepsin protease is selected from the group consisting of cathepsin K and cathepsin L.
